# EUROPEAN PATENT SPECIFICATION

(11) **EP 1 511 845 B1**
(45) Date of publication and mention of the grant of the patent: **11.01.2012**
(21) Application number: 03755959.8
(22) Date of filing: 30.05.2003
(51) Int. Cl.: C12N 15/117, A61K 31/713, A61K 39/39, A61P 37/04

(54) **IMMUNOSTIMULATORY OLIGONUCLEOTIDES AND USES THEREOF**
IMMUNOSTIMULATORISCHE OLIGONUKLEOTIDE UND DEREN VERWENDUNGEN
OLIGONUCLEOTIDES IMMUNOSTIMULATEURS ET LEUR UTILISATION

(30) Priority: 30.05.2002 CA 2388049
(43) Date of publication of application: 09.03.2005
(73) Proprietor: DAVID HORN, LLC, Doylestown PA 18902 (US)
(72) Inventor: LOPEZ, Ricardo, Augustin, Ciudad Autonoma de Buenos Aires (AR)
(74) Representative: Fernandez Lerroux, Aurelio
(86) International application number: PCT/EP2003/005691
(87) International publication number: WO 2003/101375

(56) References cited:
- WO-A-01/22972
- WO-A-01/22990
- HARTMANN G ET AL: "Mechanism and function of a newly identified CpG DNA motif in human primary B cells." JOURNAL OF IMMUNOLOGY, vol. 164, no. 2, 15 January 2000 (2000-01-15), pages 944-953, XP002276021 ISSN: 0022-1767 cited in the application
- MARIOTTI S ET AL: "Immunogenicity of anti-Haemophilus influenzae type b CRM197 conjugate following mucosal vaccination with oligodeoxynucleotide containing immunostimulatory sequences as adjuvant" VACCINE, vol. 20, no. 17-18, 22 May 2002 (2002-05-22), pages 2229-2239, XP004353929 ISSN: 0264-410X
- NEST VAN G ET AL: "AN IMMUNOSTIMULATORY OLIGONUCLEOTIDE (ISS ODN) ENHANCES IMMUNE RESPONSES TO HBV VACCINE IN A VARIETY OF ANIMAL SPECIES INCLUDING PRIMATES" ANNUAL ICAAC ABSTRACTS, vol. 39, 26 September 1999 (1999-09-26), page 374, AN679, XP001070533
- RODRIGUEZ JUAN M ET AL: 'Potent (non-CpG) immunostimulatory oligonucleotides.' FASEB JOURNAL vol. 17, no. 7, 14 April 2003, page C289, XP008029386 ISSN: 0892-6638 & 90TH ANNIVERSARY ANNUAL MEETING OF THE AMERICAN ASSOCIATION OF IMMUNOLOGISTS; DENVER, CO, USA; MAY 06-10, 2003
- ELIAS FERNANDA ET AL: "Strong cytosine-guanosine-independent immunostimulation in humans and other primates by synthetic oligodeoxynucleotides with PyNTTTTGT motifs." JOURNAL OF IMMUNOLOGY, vol. 171, no. 7, 1 October 2003 (2003-10-01), pages 3697-3704, XP002276022 ISSN: 0022-1767

## Description

### FIELD OF THE INVENTION

The present invention refers especially to oligonucleotides having up to 100 nucleotides and comprising a non-palindromic nucleic acid sequence motif having the following formula:
TCATCATTTTGTCATTTTGTCATT (SEQ ID N°2)
These oligonucleotides were shown to be immunostimulatory in animals of the order *Primate,* including humans.

### RELEVANT REFERENCES

### PATENT DOCUMENTS

US 5,663,153; US 5,723,335; US 6,090,791; US 6,194,388; US 6,207,646; US 6,239,116; US 6,429,199; US 6,544,518; US 6,514,948; US 6,498,148; US 6,429,199; US 6,426,336; US 6,406,705; US 6455689; US 20010044416; US 20020156033; US 20020165178; US 20020198165; US 2002137714; US 20030050268; US 2003040499; EP 0468520; WO 95/26204; WO 96/02555; WO 98/40100; WO 98/52962; WO 99/33488; WO 99/56755; WO 00/62802; WO 01/00231; WO 01/00232; WO 01/22972; WO 01/55370; WO 00/014217; WO 00/061151; WO 00/067023; WO 00/067787; WO 00/075304; WO 01/002007; WO 01/007055; WO 01/022990; WO 01/048018; WO 01/055341; WO 01/062909; WO 01/062910; WO 01/068077; WO 01/068116; WO 01/068143; WO 01/068144; WO 01/083503; WO 01/093905; WO 02/026757; WO 02/056909; WO 02/069369; WO 02/095027; WO 02/102825; WO 03/002065; WO 94/25588; WO 99/56755; WO 99/62923.

### OTHER REFERENCES

Ahmad-Nejad P et al., Bacterial CpG-DNA and lipopolysaccharides activate Toll-like receptors at distinct cellular compartments. Eur. J. Immunol. 32:1958-1968, (2002).
Angier N. Microbe DNA Seen as Alien By Immune System, New York Times, 11, (1995).
Auf G et al., CpG-oligodeoxynucleotide rejection of a neuroblastoma in A/J mice does not induce a paraneoplastic disease. Neurosci. Lett. 327:189-192, (2002).
Ayash-Rashkovsky M et al., Generation of Th1 immune responses to inactivated, gp120-depleted HIV-1 in mice with a dominant Th2 biased immune profile via immunostimulatory oligonucleotides--relevance to AIDS vaccines in developing countries. Vaccine. 20:2684-2692, (2002).
Azad RF et al., Antiviral Activity of a Phosphorothioate, Oligonucleotide Complementary to RNA of the Human Cytomegalovirus Major Immediate-Early Region, Antimicrobial Agents and Chemotherapy, 37:1945-1954, (1993).
Azuma, Biochemical and Immunological Studies on Cellular Components of Tubercle Bacilli. Kekkaku, 69. 9:45-55, (1992).
Ballas ZK et al., Induction of NK activity in murine and human cells by CpG motifs in oligodeoxynucleotides and bacterial DNA. J. Immunol. 157, 5:1840-1845, (1996).
Bauer M et al., Bacterial CpG-DNA triggers activation and maturation of human CD11c-, CD123+ dendritic cells. J. Immunol. 166:5000-5007 (2001).
Bauer S et al., H. Bacterial CpG-DNA licenses TLR9. Curr. Top. Microbiol. Immunol. 270:145-154 (2002).
Bauer S et al., Human TLR9 confers responsiveness to bacterial DNA via species-specific CpG motif recognition. Proc. Natl. Acad. Sci. USA, 98: 9237-9242, (2001).
Beignon AS et al., Immunization onto bare skin with synthetic peptides: immunomodulation with a CpG-containing oligodeoxynucleotide and effective priming of influenza virus-specific CD4+ T cells. Immunology, 105:204-2012 (2002).
Boggs RT et al., Characterization and modulation of immune stimulation by modified oligonucleotides. Antisense Nucleic Acid Drug Dev. 7, 5:461-471, (1997).
Bohle B. CpG motifs as possible adjuvants for the treatment of allergic diseases. Int. Arch. Allergy Immunol. 129:198-203, (2002).
Bozza S et al., Vaccination of mice against invasive aspergillosis with recombinant Aspergillus proteins and CpG oligodeoxynucleotides as adjuvants. Microbes Infect. 4:1281-1290, (2002).
Branda RF et al., Amplification of antibody production by phosphorothioate oligodeoxynucleotides. J. Lab. Clin. Med. 128, 3:329-338, (1996).
Carlson GA. Postexposure prophylaxis against transmissible spongiform encephalopathies: CpG oligodeoxynucleotides in mice. Lancet, 360:184, (2002).
Carpentier et al., Oligodeoxynucleotides containing CpG motifs can induce rejection of a neuroblastoma in mice. Cancer Res. 59, 5429-5432, (1999). Chace JH et al., Bacterial DNA-induced NK cell IFNγ production is dependent on macrophage secretion of IL-12. Clin. Immunol. Immunopathol. 84, 185-193, (1997).
Chen W et al., Enhancement of antigen-presenting ability of B lymphoma cells by immunostimulatory CpG-oligonucleotides and anti-CD40 antibody. Immunol. Lett. 77:17-23 (2001).
Choudhury BK et al., In vivo role of p38 mitogen-activated protein kinase in mediating the anti-inflammatory effects of CpG oligodeoxynucleotide in murine asthma. J. Immunol.169:5955-5961, (2002).
Chu RS et al., CpG oligodeoxynucleotides act as adjuvants that switch on T helper 1 (Th1) immunity. J. Exp. Med.186, 1623-1631, (1997).
Chuang TH et al., Toll-like receptor 9 mediates CpG-DNA signaling. J. Leukoc. Biol. 71:538-544, (2002).
Cowdery JS et al., Bacterial DNA induces NK cells to produce IFN-gamma in vivo and increases the toxicity of lipopolysaccharides. J. Immunol. 156, 12:4570-4575, (1996).
Dalpke AH et al., Phosphodiester CpG oligonucleotides as adjuvants: polyguanosine runs enhance cellular uptake and improve immunostimulative activity of phosphodiester CpG oligonucleotides in vitro and in vivo. Immunology, 106:102-1012, (2002).
Davis HL et al., CpG DNA is a potent enhancer of specific immunity in mice immunized with recombinant hepatitis B surface antigen. J. Immunol.160, 870-876, (1998).
Decker T et al., Effect of immunostimulatory CpG-oligonucleotides in chronic lymphocytic leukemia B cells. Leuk. Lymphoma. 42:301-307 (2001).
Decker T et al., Immunostimulatory CpG-oligonucleotides cause proliferation, cytokine production, and an immunogenic phenotype in chronic lymphocytic leukemia B cells. Blood, 95:999-1006 (2000).
Decker T et al., Peschel C. Immunostimulatory CpG-oligonucleotides induce functional high affinity IL-2 receptors on B-CLL cells: costimulation with IL-2 results in a highly immunogenic phenotype. Exp. Hematol. 28:558-68 (2000).
Decker T et al., Sensitization of B-cell chronic lymphocytic leukemia cells to recombinant immunotoxin by immunostimulatory phosphorothioate oligodeoxynucleotides. Blood, 99:1320-1326, (2002).
Diwan M et al., Enhancement of immune responses by co-delivery of a CpG oligodeoxynucleotide and tetanus toxoid in biodegradable nanospheres. J. Control Release. 85:247-62, (2002).
Dumais N et al., Mucosal immunization with inactivated human immunodeficiency virus plus CpG oligodeoxynucleotides induces genital immune responses and protection against intravaginal challenge. J. Infect. Dis. 186:1098-105 (2002).
Erb KJ et al., Infection of mice with Mycobacterium bovis-Bacillus Calmette-Guerin (BCG) suppresses allergen-induced airway eosinophilia. J. Exp. Med. 187, 4:561-569, (1998).
Fattorini L et al., Recombinant GroES in combination with CpG oligodeoxynucleotides protects mice against Mycobacterium avium infection. J. Med. Microbiol. 51:1071-1079, (2002).
Gierynska M et al., Induction of CD8 T-cell-specific systemic and mucosal immunity against herpes simplex virus with CpG-peptide complexes. J. Virol. 76:6568-6576 (2002).
Gill RF et al., Enhancement of rat tear IgA antibody responses following intranasal immunization with antigen and CpG ODN. Curr. Eye Res. 24:228-233, (2002).
Gursel M et al., CpG oligodeoxynucleotides induce human monocytes to mature into functional dendritic cells. Eur. J. Immunol. 32:2617-2622 (2002). Gursel M et al., Differential and competitive activation of human immune cells by distinct classes of CpG oligodeoxynucleotide. J. Leukoc. Biol. 71:813-820, (2002).
Hadden J et al., Immunopharmacology, JAMA, 268, 20:2964-2969, (1992).
Hadden J et al., Immunostimulants. TIPS, 141:169-174, (1993).
Halpern MD et al., Bacterial DNA induces murine interferon-gamma production by stimulation of interleukin-12 and tumor necrosis factor-alpha. Cell Immunol. 167, 1:72-78, (1996).
Harandi AM et al., A protective role of locally administered immunostimulatory CpG oligodeoxynucleotide in a mouse model of genital herpes infection. J. Virol. 77:953-962 (2003).
Hartmann et al., Mechanism and function of a newly identified CpG DNA motif in human primary B cells. J. Immunol.164, 944-952, (2000).
Hartmann G et al., Delineation of a CpG phosphorothioate oligodeoxynucleotide for activating primate immune responses in vitro and in vivo. J. Immunol.164, 1617-1624, (2000).
Heckelsmiller K et al., Combined dendritic cell- and CpG oligonucleotide-based immune therapy cures large murine tumors that resist chemotherapy. Eur. J. Immunol. 32:3235-45, (2002).
Heckelsmiller K et al., Peritumoral CpG DNA elicits a coordinated response of CD8 T cells and innate effectors to cure established tumors in a murine colon carcinoma model. J. Immunol. 169:3892-3899, (2002).
Horner AA et al., Immunostimulatory sequence oligodeoxynucleotide-based vaccination and immunomodulation: two unique but complementary strategies for the treatment of allergic diseases. J. Allergy Clin. Immunol. 110:706-712 (2002).
Horner AA et al., Optimized conjugation ratios lead to allergen immunostimulatory oligodeoxynucleotide conjugates with retained immunogenicity and minimal anaphylactogenicity. J. Allergy Clin. Immunol. 110:413-420, (2002).
Hornung V et al., Quantitative expression of toll-like receptor 1-10 mRNA in cellular subsets of human peripheral blood mononuclear cells and sensitivity to CpG oligodeoxynucleotides. J. Immunol. 168:4531-4537, (2002).
Hussain I et al., Modulation of murine allergic rhinosinusitis by CpG oligodeoxynucleotides. Laryngoscope, 112:1819-1826, (2002).
Ioannou XP et al., CpG-containing oligodeoxynucleotides, in combination with conventional adjuvants, enhance the magnitude and change the bias of the immune responses to a herpesvirus glycoprotein. Vaccine 21:127-37, (2002). Ioannou XP et al., The immunogenicity and protective efficacy of bovine herpesvirus 1 glycoprotein D plus Emulsigen are increased by formulation with CpG oligodeoxynucleotides. J. Virol. 76:9002-9010, (2002).
Ishii KJ, et al., Potential role of phosphatidylinositol 3 kinase, rather than DNA-dependent protein kinase, in CpG DNA-induced immune activation. J. Exp. Med. 196:269-274 (2002).
Jahrsdorfer B et al., CpG DNA increases primary malignant B cell expression of costimulatory molecules and target antigens. J. Leukoc. Biol. 69:81-88 (2001).
Jahrsdorfer B et al., Modulation of malignant B cell activation and apoptosis by bcl-2 antisense ODN and immunostimulatory CpG ODN. J. Leukoc. Biol. 72:83-92, (2002).
Jain VV et al., CpG-oligodeoxynucleotides inhibit airway remodeling in a murine model of chronic asthma. J. Allergy Clin. Immunol. 110:867-872, (2002).
Jiang W et al., Mucosal immunization with helicobacter, CpG DNA, and cholera toxin is protective. Infect. Immun. 71:40-46, (2003).
Joseph A et al., Liposomal immunostimulatory DNA sequence (ISS-ODN): an efficient parenteral and mucosal adjuvant for influenza and hepatitis B vaccines. Vaccine. 20:3342-3354 (2002).
Juffermans NP et al., CpG oligodeoxynucleotides enhance host defense during murine tuberculosis. Infect. Immun. 70:147-152 (2002).
Jung J et al., Distinct response of human B cell subpopulations in recognition of an innate immune signal, CpG DNA. J. Immunol. 169:2368-2373, (2002).
Kadowaki N et al., Distinct CpG DNA and polyinosinic-polycytidylic acid double-stranded RNA, respectively, stimulate CD11c- type 2 dendritic cell precursors and CD11c+ dendritic cells to produce type I IFN. J. Immunol., 166, 2291- 2295, (2001).
Kadowaki N et al., Subsets of human dendritic cell precursors express different toll-like receptors and respond to different microbial antigens. J. Exp. Med. 17;194:863-869 (2001).
Kataoka T et al., Antitumor Activity of Synthetic Oligonucleotides with Sequences from cDNA Encoding Proteins of Mycobacterium bovis BCG. Jpn. J. Cancer Res., 83:244-247, (1992).
Kimura T et al., Binding of Oligoguanylate to Scavenger Receptors Is Requiered for Oligonucleotides to Augment NK Cell Activity and Induce IFN. J. Biochem. 116, 5:991-994, (1994).
Kitagaki K et al., Immunomodulatory effects of CpG oligodeoxynucleotides on established th2 responses. Clin. Diagn. Lab. Immunol. 9:1260-1269, (2002).
Kline JN et al., CpG oligonucleotides can reverse as well as prevent TH2-mediated inflammation in a murine model of asthma. J. Invest. Med., 45, 7:298A, (1997).
Kline JN et al., Immune redirection by CpG oligonucleotides. Conversion of a Th2 response to a Th1 response in a murine model of asthma. J. Invest. Med., 45, 3:282A, (1997).
Kline JN et al., Modulation of airway inflammation by CpG oligodeoxynucleotides in a murine model of asthma. J. immunol. 15, 2555-2559, (1998).
Kline JN et al., Treatment of established asthma in a murine model using CpG oligodeoxynucleotides. Am. J. Physiol. Lung. Cell. Mol. Physiol. 283:170-179, (2002).
Klinman DM et al., CpG motifs present in bacteria DNA rapidly induce lymphocytes to secrete interleukin 6, interleukin 12, and interferon gamma. Proc. Natl. Acad. Sci. USA, 93, 7:2879-2883, (1996).
Krieg AM el all A Role for Endogenous Retroviral Sequences in the Regulation of Lymphocyte Activation. J. Immunol., 143, 2448-2451, (1989).
Krieg AM et al, Phosphorothioate Oligodeoxynucleotides: Antisense or Anti-Protein?, Antisense Res. Develop. 5:241, (1995).
Krieg AM et al. Leukocyte Stimulation by Oligodeoxynucleotides. Applied Antisense Oligonucleotide Technology, 431-448, (1998).
Krieg AM et al., CpG motifs in bacterial DNA trigger direct B-cell activation. Nation, 374:546-549, (1995).
Krieg AM et al., Oligodeoxynucleotide modifications determine the magnitude of B cell stimulation by CpG motifs. Antisense Nucleic Acid Drug Dev., 6, 2:133-139, (1996).
Krieg AM et al., The role of CpG dinucleotides in DNA vaccines. Trends in Microbiology, 6, 23-37, (1998).
Krieg AM, An innate immune defense mechanism based on the recognition of CpG motifs in microbial DNA. J. Lab. Clin. Med., 128, 2:128-33, (1996).
Krieg AM. CpG motifs in bacterial DNA and their immune effects. Annu. Rev. Immunol. 20:709-760 (2002).
Krieg AM. GpG motifs in bacterial DNA and their immune effects. Annu. Rev. Immunol., 20, 709-760, (2002).
Krug A et al., Identification of CpG oligonucleotide sequences with high induction of IFN-alpha/beta in plasmacytoid dendritic cells. Eur. J. Immunol. 31:2154-2163 (2001).
Krug A et al., Toll-like receptor expression reveals CpG DNA as a unique microbial stimulus for plasmacytoid dendritic cells which synergizes with CD40 ligand to induce high amounts of IL-12. Eur. J. Immunol. 31:3026-3037, (2002).
Kuramoto, et al., Oligonucleotide Sequences Required for Natural Killer Cell Activation. Jpn. J. Cancer Res. 83:1128-1131, (1992).
Liang H et al., Activation of human B cells by phosphorothioate oligodeoxynucleotides. J. Clin. Invest. 98, 1119-1129, (1996).
Lingnau K et al., Poly-L-arginine synergizes with oligodeoxynucleotides containing CpG-motifs (CpG-ODN) for enhanced and prolonged immune responses and prevents the CpG-ODN-induced systemic release of proinflammatory cytokines. Vaccine. 20:3498-3508, (2002).
Lipford GB et al., CpG-containing synthetic oligonucleotides promote B and cytotoxic T cell responses to protein antigen: a new class of vaccine adjuvants. Eur. J. Immunol., 27:2340-2344, (1997).
Lipford GB et al., Immunostimulatory DNA: sequence-dependent production of potentially harmful or useful cytokines. Eur. J. Immunol. 27:3420-3426, (1997).
Macfarlane DE and Manzel L, Antagonism of immunostimulatory CpG-oligodeoxynucleotides by quinacrine, chloroquine, and structurally related compounds. J. Immunol., 160, 3:1122-1131, (1998).
Magone MT et al., Systemic or mucosal administration of immunostimulatory DNA inhibits early and late phases of murine allergic conjunctivitis. Eur. J. Immunol. 30, 1841-1850, (2000).
Mariotti S et al., Immunogenicity of anti-Haemophilus influenzae type b CRM197 conjugate following mucosal vaccination with oligodeoxynucleotide containing immunostimulatory sequences as adjuvant. Vaccine. 20:2229-2239, (2002).
Matson S et al., Nonspecific suppression of [3H]thymidine incorporation by "control" oligonucleotides. Antisense Res. Dev., 2, 4:325-330, (1992).
Maurer T et al., CpG-DNA aided cross-presentation of soluble antigens by dendritic cells. Eur. J. Immunol. 32:2356-2364.
McCluskie MJ et al., Parenteral and mucosal prime-boost immunization strategies in mice with hepatitis B surface antigen and CpG DNA. FEMS Immunol. Med. Microbiol. 32:179-185, (2002).
Messina et al., Stimulation of in vitro Murine Lymphocyte Proliferation by Bacterial DNA. J. Immunol., 147, 6:1759-1764, (1991).
Messina, et al., The Influence of DNA Structure on the in vitro Stimulation of Murine Lymphocytes by Natural and Synthetic Polynucleotide Antigens. Cell. Immunol., 147:148-157, (1993).
Miconnet I et al., CpG are efficient adjuvants for specific CTL induction against tumor antigen-derived peptide. J. Immunol. 168:1212-1218 (2002). Mojcik C et al., Administration of a Phosphorothioate Oligonucleotide Antisense Murine Endogenous Retroviral MCF env Causes Immune Effect in vivo in a Sequence-Specific Manner. Clin. Immunol. Immunopathology. 67, 2:130-136, (1993).
Moldovenu GJ et al., CpG DNA, a novel immune enhancer for systemic and mucosal immunization with influenza virus. Vaccine.16, 1216-1224, (1998). O'Hagan DT et al. Synergistic adjuvant activity of immunostimulatory DNA and oil/water emulsions for immunization with HIV p55 gag antigen. Vaccine. 20:3389-98, (2002).
Olbrich AR et al., Effective postexposure treatment of retrovirus-induced disease with immunostimulatory DNA containing CpG motifs. J. Virol. 76:11397-404, (2002).
Pal S et al., Immunization with the Chlamydia trachomatis mouse pneumonitis major outer membrane protein by use of CpG oligodeoxynucleotides as an adjuvant induces a protective immune response against an intranasal chlamydial challenge. Infect. Immun. 70:4812-4817, (2002).
Park Y et al., Cutting Edge: CpG DNA inhibits dendritic cell apoptosis by up-regulating cellular inhibitor of apoptosis proteins through the phosphatidylinositide-3'-OH kinase pathway. J. Immunol. 168:5-8 (2002).
Pisetsky et al., Stimulation of Murine Lymphocyte Proliferation by a Phosphorothioate Oligonucleotide with Antisense Activity for Herpes Simplex Virus. Life Science, 54, 101-107 (1994).
Pisetsky, Immunological Consequences of Nucleic Acid Therapy. Antisense Res. Development, 5:219-225 (1995).
Pisetsky, The Immunological Properties of DNA. J. Immunol., 421-423 (1996).
Rachmilewitz D et al., Immunostimulatory DNA ameliorates experimental and spontaneous murine colitis. Gastroenterology. 122:1428-1441, (2002).
Rankin R et al., CpG motif identification for veterinary and laboratory species demonstrates that sequence recognition is highly concerved. Antisence Nucleic Acid Drug Dev., 11:333-340 (2001).
Rankin R et al., CpG-containing oligodeoxynucleotides augment and switch the immune responses of cattle to bovine herpesvirus-1 glycoprotein D. Vaccine, 20:3014-3022, (2002).
Rhee EG et al., Vaccination with heat-killed leishmania antigen or recombinant leishmanial protein and CpG oligodeoxynucleotides induces long-term memory CD4+ and CD8+ T cell responses and protection against leishmania major infection. J. Exp. Med. 195:1565-1573 (2002).
Rothenfusser S et al., Plasmacytoid dendritic cells: the key to CpG. Human Immunol. 63:1111-1119, (2002).
Santeliz JV et al., Amb a 1-linked CpG oligodeoxynucleotides reverse established airway hyperresponsiveness in a murine model of asthma. J. Allergy Clin. Immunol. 109:455-462, (2002).
Sato et al., Immunostimulatory DNA Sequences Necessary for Effective Intradermal Gene Immunization. Science, 273:352-354, (1996).
Schultz KR et al., Chloroquine prevention of murine MHC-disparate acute graft-versus-host disease correlates with inhibition of splenic response to CpG oligodeoxynucleotides and alterations in T-cell cytokine production. Biol. Blood Marrow Transplant, 8:648-655 (2002).
Schwartz DA et al., CpG motifs in bacterial DNA cause inflammation in the lower respiratory tract. J. Clin. Invest., 100, 1:68-73, (1997).
Senuma A et al., Therapeutic effect of CpG motifs on the development of chronic graft-versus-host disease in mice. Cytokine. 20:23-29, (2002).
Sethi S et al., Postexposure prophylaxis against prion disease with a stimulator of innate immunity. Lancet, 360:229-230, (2002).
Sfondrini L et al., Absence of the CD1 molecule up-regulates antitumor activity induced by CpG oligodeoxynucleotides in mice. J. Immunol. 169:151-158, (2002).
Shirota H et al., B cells capturing antigen conjugated with CpG oligodeoxynucleotides induce Th1 cells by elaborating IL-12. J. Immunol. 169:787-94, (2002).
Sparwasser T et al., Bacterial DNA and immunostimulatory CpG oligodeoxynucleotides trigger maturation and activation of murine dendritic cells. Eur. J. Immunol. 28, 2045-2054, 1998.
Stern BV et al., Vaccination with tumor peptide in CpG adjuvant protects via IFN-gamma-dependent CD4 cell, immunity. J. Immunol. 168:6099-6105, (2002).
Sweet MJ et al., Colony-stimulating factor-1 suppresses responses to CpG DNA and expression of toll-like receptor 9 but enhances responses to lipopolysaccharide in murine macrophages. J. Immunol. 168:392-399 (2002). Takauji R et al., CpG-DNA-induced IFN-alpha production involves p38 MAPK-dependent STAT1 phosphorylation in human plasmacytoid dendritic cell precursors. J. Leukoc. Biol. 72:1011-1019 (2002).
Takeshita F et al., Cutting edge: Role of Toll-like receptor 9 in CpG DNA-induced activation of human cells. J. Immunol. 67:3555-3558, (2001). Tokunaga T et al. Synthetic Oligonucleotides with Particular Base Sequences from the cDNA Encoding Proteins of Mycobacterium bovis BCG Induce Interferons and Activate Natural Killer Cells. Microbiol. Immunol., 36, 1:55-66, (1992).
Tokunaga T et al., A synthetic single-stranded DNA, poly(dG,dC), induces interferon-alpha/beta and -gamma, augments natural killer activity, and suppresses tumor growth. Jpn. J. Cancer Res., 79:682-686, (1988).
Van der Stede Y et al., CpG-oligodinucleotides as an effective adjuvant in pigs for intramuscular immunizations. Vet. Immunol. Immunopathol. 86:31-41, (2002).
Verthelyi D et al., CpG oligodeoxynucleotides as vaccine adjuvants in primates. J. Immunol. 168:1659-1663 (2002).
Verthelyi D et al., Human peripheral blood cells differentially recognize and respond to two distinct CPG motifs. J. Immunol. 166: 2372-2377, (2001). Vicari AP et al., Reversal of tumor-induced dendritic cell paralysis by CpG immunostimulatory oligonucleotide and anti-interleukin 10 receptor antibody. J. Exp. Med. 196:541-549, (2002).
Vollmer J et al., Highly immunostimulatory CpG-free oligodeoxynucleotides for activation of human leukocytes. Antisense Nucleic Acid Drug Dev.12:165-175, (2002).
Warren TL and Weiner GJ. Synergism between cytosine-guanine oligodeoxynucleotides and monoclonal antibody in the treatment of lymphoma. Semin. Oncology, 29:93-97 (2002).
Weeratna R et al., CpG DNA induces stronger immune responses with less toxicity than other adjuvants. Vaccine, 18, 1755-1762, (2000).
Wernette CM et al., CpG oligodeoxynucleotides stimulate canine and feline immune cell proliferation. Vet. Immunol. Immunopathol. 84:223-236 (2002). Wooldridge JE et al., Immunostimulatory oligodeoxynucleotides containing CpG motifs enhance the efficacy of monoclonal antibody therapy of lymphoma. Blood, 89, 2994-2998, (1997).
Yamamoto S et al., In vitro augmentation of natural killer cell activity and production of interferon alpha/beta and gamma with deoxyribonucleic acid fraction from Mycobacterium bovis BCG. Jpn. J. Cancer Res., 79:866-873, (1988).
Yamamoto S. et al., DNA from Bacteria, but Not from Vertebrates, Induces Interferons, Activates Natural Killer Cells, and Inhibits Tumor Growth. Microbiol. Immunol. 36, 9:983-997, (1992).
Yamamoto S. et al., Unique Palindromic Sequences in Synthetic Oligonucleotides are Required to Induce INF and Augment INF-Mediated Natural Killer Activity. J. Immunol., 148, 12:4072-4076, (1992).
Yamamoto S., Mode of Action Of Oligonucleotide Faction Extracted From Mycobacterium bovis BCG. Kekkaku, 69, 9:29-32, (1994).
Yamamoto T et al., Ability of Oligonucleotides with Certain Palindromes to Induce Interferon Produtin and Augment Natural Killer Cell Activity Is Associated with Their Base Length. Antisense Res. Devel., 4:119-123, (1994).
Yamamoto T et al., Lipofection of Synthetic Oligodeoxyribonucleotide Having a Palindromic Sequence AACGTT to Murine Splenocytes Enhances Interferon Production and Natural Killer Activity. Microbiol. Immunol. 38, 10:831-836, (1994).
Yamamoto T et al., Synthetic Oligonucleotides with Certain Palindromes Stimulate Interferon Production Of Human Peripheric B Lymphocytes in vitro, Jpn. J. Cancer Res., 85:775-779, (1994).
Yi A et al., IFN-.gamma. Promotes IL-6 and IgM Secretion in Response to CpG Motifs in Bacterial DNA and Oligonucleotides. J. Immunol. 558-564 (1996).
Yi AK et al. Role of mitogen-activated protein kinases in CpG DNA-mediated IL-10 and IL-12 production: central role of extracellular signal-regulated kinase in the negative feedback loop of the CpG DNA-mediated Th1 response. J. Immunol. 168:4711-4720, (2002).
Yi Ae-Kyung et al., Rapid Immune Activation by CpG Motifs in Bacterial DNA. J. Immunol., 5394-5402 (1996).
Yu D et al., Immunomers novel 3'-3'-linked CpG oligodeoxyribonucleotides as potent immunomodulatory agents. Nucleic Acids Res. 30:4460-4469. (2002). Zelenay, S. Et al., Immunomodulatory effects of plasmid DNA and synthetic oligodeoxynucleotides. J. Eur. J. Immunol. 33: 1382-92 (2003)
Bylund, et al. European Journal of Immunology (2002), 32(10), 2847-2856 Dalpke, et al. Immunology (2002), 106(1), 102-112
Kandimalla, et al. Bioorganic & Medicinal Chemistry (2001), 9(3), 807-813
Lang, et al. European Journal of Immunology (1999), 29(11), 3496-3506
McCluskie, et al. Vaccine (2001), 19(17-19), 2657-2660
Monteith, et al. Anti-Cancer Drug Design (1997), 12(5), 421-432
Oumouna, et al. Developmental & Comparative Immunology (2002), 26(3), 257-269
Parronchi, et. al. Journal of Immunology (1999), 163(11), 5946-5953 Vollmer, et al. Antisense & Nucleic Acid Drug Development (2002), 12(3), 165-175
Zhao, et al. Biochemical Pharmacology (1996), 51 (2), 173-82

### BACKGROUND OF THE INVENTION

### The immune system

The major function of the immune system is to protect the host from invading pathogens. A number of different cell types, both antigen-independent and antigen-specific, have evolved to detect and neutralize these invading pathogens. Among them, lymphocytes have an important characteristic, which is their ability to specifically recognize antigens, a feature not possessed by any other cell. This means that any lymphocyte function stimulated by an antigen is directed solely at that antigen. Lymphocytes may be divided into two major populations: T and B. T-lymphocytes have a central role in regulating the immune response and for this they produce and secrete lymphokines (i.e.interleukins). On the other hand, B-lymphocytes are the only cells that produce antibodies, which are proteins that recognize and bind antigens.

Some T-lymphocytes are known as helpers (Th-lymphocytes) because they assist B-cells to produce antibodies. T-lymphocytes express a characteristic membrane molecule designated CD4. Other T-lymphocytes are known as cytotoxic (CTL) because they are capable of killing certain cells. They express a different characteristic membrane protein designated CD8.

In mice, Th-lymphocytes have been subdivided in groups which are designated Th0, Th1 and Th2, according to the lymphokines they produce. In general, Th1 lymphocytes produce lymphokines which stimulate macrophages and CTLS (IL2, IFN γ, TNF-β); Th2 lymphocytes produce lymphokines which stimulate B-lymphocytes to proliferate and to produce antibodies (IL 2, IL5, IL6, IL10, IL13); and Th0 lymphocytes produce a mixture of lymphokines and are thought to be an intermediate stage from which Th1 and Th2 lymphocytes are derived. In humans, Th1- and Th2- like lymphocytes have been demonstrated, although they seem to show a less strict division with respect to their patterns of cytokine secretion.

A third population of lymphocytes, which lack the major makers of T and B cells include the natural killer cells (NK cells), the killer cells (K cells) and the lymphokine-activated killer cells (L A K cells). NK cells can kill certain tumor cells and some virally infected cells, but unlike cytotoxic T-lymphocytes, they are not capable of recognizing a specific antigen, First, they can bore a hole in a target cell by secreting perforin molecules to form a membrane attack complex on the surface of the target. NK cells can then secrete enzymes that enter the target cell and cause it to commit suicide. As a second mechanism of destruction, Fas ligand, a protein present on NK cells can interact with Fas, another protein present on the surface of the target cell, inducing the target cell to commit suicide by apoptosis. NK cells are also able to bind to cells, which have antibody to them via their antigen-binding regions and kill them. L A K cells do not specifically recognize an antigen but they are capable of destroying a wider range of targets than NK cells.

Macrophages and dendritic cells play a critical role in initiating immune responses, helping T cells to respond to antigens.

There are several classes of antibody. The IgG class comprises most of the circulating antibodies and it has four subclasses designated IgG1, IgG2, IgG3 and IgG4. The IgM class comprises about 10 % of the circulating antibodies. These are the principal antibodies produced during the primary immunological response. The IgA class comprises most of the antibodies secreted at mucous membranes and exerts its protective effect by blocking access of the antigen to the inner body. The IgD class comprises less than 1 % of serum antibodies and its biological role is largely unknown. The IgE class comprises antibodies that are mainly bound to the surface of most cells and basophils. These antibodies are associated with reactions that occur in individuals who are undergoing allergic reactions.

### Vaccines and vaccine adjuvants

Vaccines are preparations used to stimulate animals to mount an immune response against antigens included in the vaccine.

Vaccines often include adjuvants, which are substances that used in combination with specific antigen produce more immunity than the antigen used alone. (Ramon, G.,1926. Procedes pour accroite la production des antitoxins. Ann. Inst. Pasteur. 40, 1- 10).

Many kinds of compounds function as vaccine adjuvants (Edelman, R. , 2000. An overview of adjuvant use, in : Vaccine Adjuvants. Preparation Methods and Research Protocols. D.T. O' Hagan, Ed., Humana Press, Totowa, New Jersey. References cited in this article are incorporated herein as background material).

However, currently, the only adjuvants approved for use in humans are aluminum salts (Gupta, R.K. and Rost, B.E., 2000. Aluminum compounds as vaccine adjuvants in: Vaccine Adjuvants. Preparation Methods and Research Protocols. D.T. O' Hagan, Ed., Humana Press, Totowa, New Jersey) and the oil-in-water emulsion M F 59 (Ott, G. Radhakrishman, R. Fang, J. and Hora, M., 2000. The adjuvant M F 59: A 10- Year Perspective, in : Vaccine Adjuvants. Preparation Methods and Research Protocols. D.T. O' Hagan, Ed., Humana Press, Totowa, New Jersey).

### Nucleic acids as immunostimulatory compounds

Several polynucleotides have been demonstrated to have immunostimulatory properties. For example, poly (I,C) is an inducer of interferon (IFN) production, macrophage activation and NK cell activation (Talmadge, J.E., Adams, J., Phillips, H., Collins, M., Lenz, B., Schneider, M., Schlick, E., Ruffmann, R., Wiltrout, R.H., Chirigos, M.A.1985. Immunomodulatory effects in mice of polyinosinic-polycytidylic acid complexed with poly-L:-lysine and carboxymethylcellulose. Cancer Res. 45:1058; Wiltrout, R.H., Salup, R.R., Twilley, T.A., Talmage, J.E. 1985. Immunomodulation of natural killer activity by polyribonucleotides. J. Biol. Resp. Mod. 4:512), poly (dG,dC) is mitogenic for B cells (Messina, J.P., Gilkerson, G.S., Pisetsky, D.S. 1993. The influence of DNA structure on the in vitro stimulation of murine lymphocytes by natural and synthetic polynucleotide antigens. Cell. Immunol. 147:148) and induces IFN and NK activity (Tocunaga, T., Yamamoto, S., Namba, K.1988. A synthetic single-stranded DNA, poly(dG,dC), induces interferon-α/β and -γ, augments natural killer activity, and suppresses tumor growth. Jpn. J. Cancer Res. 79:682).

Bacterial DNA has also been reported to have immunostimulatory properties. These properties include the induction of cytokines (interferon gamma (IFN γ), alpha (IFN α) and beta (IFN β)), tumor necrosis factor alpha (TNF α), interleukin 6 (IL6), 12 (IL 12) and 18 (IL 18), as well as the direct stimulation of B cells (Yamamoto, S. et al. 1988. In vitro augmentation of natural killer cell activity of interferon α/β and γ with deoxyribonucleic acid fraction from Mycobacterium bovis BCG. Jpn. J. Cancer Res. (Gann) 79: 866-873; Yamamoto S. et al., 1992. DNA from bacteria, but not from vertebrates, induces interferons, activates natural killer cells and inhibits tumor growth. Microbiol. Immunol. 36: 983-997; Klinman, D.M., Yi, A-K., Beaucage, S.L., Conover, J. and Krieg, A.M.,1996. CpG motifs present in bacterial DNA rapidly induce lymphocytes to secrete interleukin 6, interleukin 12 and interferon γ. Proc. Natl. Acad. Sci. USA 93, 2879-2883. Halpern, M. D., et al. 1996. Bacterial DNA induces murine interferon -γ production by stimulation of interleukin -12 and tumor necrosis factor -α. Cell. Immunol. 167: 72-78. Sparwasser, T. et al., 1997. Macrophages sense pathogens via DNA motifs: induction of tumor necrosis factor -α - mediated shock. Eur. J. Immunol. 27: 1671-1679; Krieg, A.M. et al., 1995. CpG motifs in bacterial DNA trigger direct B-cell activation. Nature 374: 345-349).

In contrast, it has been reported that mammalian DNA has no significant immune effects (Pisetsky, D.S. 1996. The immunologic properties of DNA. J. Immunol. 156: 421-423; Messina et al. 1991. Stimulation of in vitro murine lymphocyte proliferation by bacterial DNA. J. Immunol. 147: 1759).

Synthetic DNA has also been reported to be immunostimulatory if it contains unmethylated CpG motifs. (Yamamoto, S et al.; 1992. Unique palindromic sequences in synthetic oligonucleotides are required to induce INF and augment INF-mediated natural killer activity. J. Immunol. 148: 4072-4076; Ballas, Z.K., et al.; 1996. Induction of NK activity in murine and human cells by CpG motifs in oligodeoxynucleotides and bacterial DNA. J. Immunol. 157: 1840-1845; Hartmann, G., Krieg, A.M. 2000. Mechanism and function of a newly identified CpG DNA motif in human primary B cells. J. Immunol. 164:944; Hartmann, G., Weeratna, R.D., Ballas, Z.K., Payette, P., Blackwell, S., Suparto, I., Rasmussen, W.L., Waldschmidt, M., Sajuthi, D., Purcell, R.H., Davis, H.L., Krieg, A.M. 2000. Delineation of a CpG phosphorothioate oligodeoxynucleotide for activating primate immune responses in vitro and in vivo. J. Immunol. 164:1617; Verthelyi, D., Ishii, K.J., Gursel, M., Takeshita, F., Klinman, D.M. 2001. Human peripheric blood cells differentially recognize and respond to two distinct CpG motifs. J. Immunol. 166:2372). However, one oligonucleotide containing phosphorothioate bonds that lack CpG motifs has been found to have some immunostimulatory activity on human B cells (Liang, H., Nishioka, Y., Reich, C.F., Pisetsky, D.S., Lipsky, P.E. 1996. Activation of human B cells by phosphorothioate oligonucleotides. J. Clin. Invest. 98:1119). This particular non-CpG oligonucleotide containing phosphorothioate bonds is a poly-T chain, 20 nucleotides long. Also, Vollmer et al (Vollmer J, Janosch A, Laucht M, Ballas ZK, Schetter C, Krieg AM. Highly immunostimulatory CpG-free oligodeoxynucleotides for activation of human leukocytes. Antisense Nucleic Acid Drug Dev.12:165-175, 2002) reported immunostimulation by phosphorothioate poly-T ODNs. These authors pointed out that poly-T ODNs are only active as phosphorothioate ODNs and have much lower activity than CpG ODNs.

It has now been discovered that non-CpG oligonucleotides containing the following non-palindromic sequence motif:

X₁X₂X₃X₄X₅X₆X₇X₈,

wherein X₁ is C or T, X₂ is C, T, G or A, X₃ is T or A, X₄ is T, C or G, X₅ is T, C or G, X₆ is T or G, X₇ is G and X₈ is T and wherein C never precedes G (in other terms, the nucleic acid motif does not consist of a CpG oligonucleotides) have potent immunostimulatory activity in animals of the order *Primate* including humans. Therefore, these oligonucleotides can be administered to subjects to treat "immune system deficiencies" or used as adjuvants, in conjunction with a vaccine, to boost the immune system in order to have a better response to the vaccine. They can also be administered to subjects to increase their responsiveness to tumors.

### SUMMARY OF THE INVENTION

It has now been discovered that non-CpG oligonucleotides containing the following non-palindromic sequence motif:

**X₁X₂X₃X₄X₅X₆X₇X₈,**

wherein X₁ is C or T, X₂ is C, T, G or A, X₃ is T or A, X₄ is T, C or G, X₅ is T, C or G, X₆ is T or G, X₇ is G and X₈ is T and wherein at least two of X₃, X₄, X₅ and X₆ are Ts and wherein C never precedes G (in other terms, the nucleic acid motif does not consist of a CpG oligonucleotides), have the ability to stimulate the immune response of animals of the order *Primate,* including humans. According to a preferred embodiment, X₁ consists of a C. It is preferable if X₃,X₄,X₅,X₆ X₇ X₈ of the immunostimulatory motif consists of TTTTGT, ATTTGT or ATTGGT. It is even more advantageous if X₁ is C; X₃ is T or A; X4 is T; X5 is T; X6 is T or G; X7 is G and X8 is T.

The oligonucleotides of this invention are useful as adjuvants in a vaccine formulation comprising one or more antigens. In embodiments of this aspect, the vaccine formulation can be liquid or lyophilized in dosage form. Many dosage forms are known in the art and can be applied herein. In embodiments of this aspect the oligonucleotides of this invention are present in the composition at a dose of from about 10 to 10,000 µg per dose. In these preparations, the oligonucleotides of this invention may be combined with other immunostimulant compounds. Examples of well-known immunostimulants are: α-interferon, β-interferon, γ-interferon, granulocyte macrophage colony stimulator factor (GM-CSF), interleukin 2 (IL2), interleukin 12 (IL12) and CpG oligonucleotide.

In preferred embodiments, the antigenic component of the vaccine is one or more antigens, either natural or recombinant, of viruses like: Human immunodeficiency viruses, such as HIV-1 and HIV-2, polio viruses, hepatitis A virus, human coxsackie viruses, rhinoviruses, echoviruses, equine encephalitis viruses, rubella viruses, dengue viruses, encephalitis viruses, yellow fever viruses, coronaviruses, vesicular stomatitis viruses, rabies viruses, ebola viruses, parainfluenza viruses, mumps virus, measles virus, respiratory syncytial virus, influenza viruses, Hantaan viruses, bunga viruses, hemorrhagic fever viruses, reoviruses, orbiviuises, rotaviruses, Hepatitis B virus, parvoviruses, papilloma viruses, polyoma viruses, adenoviruses, herpes simplex virus (HSV) 1 and 2, varicella zoster virus, cytomegalovirus (CMV), variola viruses, vaccinia viruses, pox viruses, African swine fever virus, the unclassified agent of delta hepatitis, the agents of non-A, non-B hepatitis; or one or more antigens of infectious bacteria like: *Helicobacter pylori, Borrelia burgdorferi, Legionella pneumophila, Mycobacterium tuberculosis, Mycobacterium bovis (BCG), Mycobacterium avium, Mycobacterium intracellulare, Staphylococcus aureus, Neisseria gonorrhoeae, Neisseria meningitidis, Listeria monocytogenes, Streptococcus pyogenes, Streptococcus pneumoniae, Haemophilus influenzae, Moraxella catharralis, Klebsiella pneumoniae, Bacillus anthracis, Corynebacterium diphtheriae, Clostridium perfringers, Clostridium tetani, Enterobacter aerogenes, Klebsiella pneumoniae, Pasturella multocida, and Treponema pallidum;* of infectious fungi like: *Cryptococcus neoformans, Histoplasma capsulatum, Coccidioides immitis, Blastomyces dermatitidis, Candida albicans;* of infectious protists like: *Plasmodium falciparum, Trypanosoma cruzi, Leishmania donovani* and *Toxoplasma gondii.* and; of human tumoral cells.

In embodiments of this aspect, one or more of the oligonucleotides of this invention and the antigen are administered simultaneously locally (by oral, rectal, intranasal or transdermal route) or systemically (by intradermic or intramuscular injection).

An aspect of this invention is the use of the present oligonucleotides for the manufacture of a medicament for vaccinating a person. The person can be vaccinated *prophylactically* or *therapeutically.*

*A prophylactic* vaccine is designed to elicit protection from a disease caused by an infectious agent through the induction of specific immunity.

*A therapeutic* vaccine is designed to induce remission of an illness (i.e. a tumor and metastasis or illness associated with an infectious agent like the human immunodeficiency virus).

The method of vaccination includes administering one or more of the oligonucleotides of this invention and one or more antigens - that is, the vaccine can be designed against one disease target or a combination of disease targets.

Another aspect of this invention is a method of treatment of a person with a tumoral disease or an immunological disorder, namely the use of one or more of the oligonucleotides of this invention for the manufacture of a medicament to stimulate his/her endogenous immune response. Examples of tumoral disease are: Chronic Myelogenous Leukemia, Precursor B-lymphoblastic lymphoma, B-cell chronic lymphocytic leukaemia, Lymphoplasmacytic lymphoma, Mantle cell lymphoma, Follicle center lymphoma, (follicular and diffuse), Marginal zone-B lymphoma, Extranodal lymphoma, Nodal marginal zone B-cell lymphoma, Splenic marginal zone B-cell lymphoma, Hairy cell leukaemia, Plasmocytoma, Diffuse large B-cell lymphoma, Burkitt's lymphoma, High grade B-cell lymphoma, Burkitt like, Melanoma, Kaposi's Sarcoma, Multiple Myeloma, Renal Cell Carcinoma, Bladder Cancer, Lung Cancer, Skin Cancer, Breast Cancer, Colon Cancer and Uterus Cancer. Examples of immunological disorders are: Allergy, Severe Combined Immunodeficiency, Chronic Granulomatous disease, and Acquired Immunodeficiency Disease.

In embodiments of this aspect, one or more of the oligonucleotides of this invention are present in a pharmaceutical formulation that can be liquid or lyophilized in dosage form. Many dosage forms are known in the art and can be applied herein. In embodiments of this aspect one or more of the oligonucleotides of this invention are present in the composition at a dose of from about 10 to 10,000 µg per dose. In these preparations one or more of the oligonucleotides of this invention may be combined with other immunostimulant compounds. Examples of well-known immunostimulants are: α-interferon, β-interferon, γ-interferon, granulocyte macrophage colony stimulator factor (GM-CSF), interleukin 2 (IL2), interleukin 12 (IL12), CpG oligonucleotides and *Mycobacterium bovis* BCG cells. Also, one or more of the oligonucleotides of this invention may be combined with an antiinfective or anticancer drug, or a surgical procedure. In all these cases, the oligonucleotides of this invention may be administered before, after or simultaneously with the alternative treatment.

Another aspect of this invention is that a person with a tumoral disease or an immunological disorder could be treated by contacting lymphocytes or plasmacytoid dendritic cells from the subject, with one or more of the oligonucleotides of this invention "ex vivo" and readministering the activated cells to the subject. In embodiments of this aspect one or more of the oligonucleotides of this invention are present in the incubation media in a concentration of about 0.10 to 100 µg per ml.

### BRIEF DESCRIPTION OF THE FIGURES

Fig. 1 is a graph plotting the proliferation index of human peripheral blood mononuclear leukocytes (PBMC) incubated with the phosphorothioate ODN IMT 023 (Seq. ID N° 9) or the phosphorothioate CpG ODN 2006: 5' TCGTCGTTTTGTCGTTTTGTCGTT 3' or the phosphorothioate non-CpG ODN IMT 021 (SEQ ID NO:1). Data represent the mean and standard deviation of five independent assays.
Fig 2 shows the results from a flow-cytometric study using human peripheral blood mononuclear leukocytes (PBMC) to determine the comparative effect of the phosphorothioate CpG ODN 2006:
   5' TCGTCGTTTTGTCGTTTTGTCGTT 3'
   or the phosphorothioate non-CpG ODN IMT 021 (SEQ ID NO:1) on activation of the B cell population. B cell activation was measured by the induction of CD86 on CD69 positive cells.
   (a) Representative flow-cytometric diagrams comparing B cell activation by ODNs IMT023, (Seq. ID N° 9), 2006 or IMT021 (SEQ ID NO:1); b)
   Representative histograms of the flow-cytometric diagrams showed in (a).

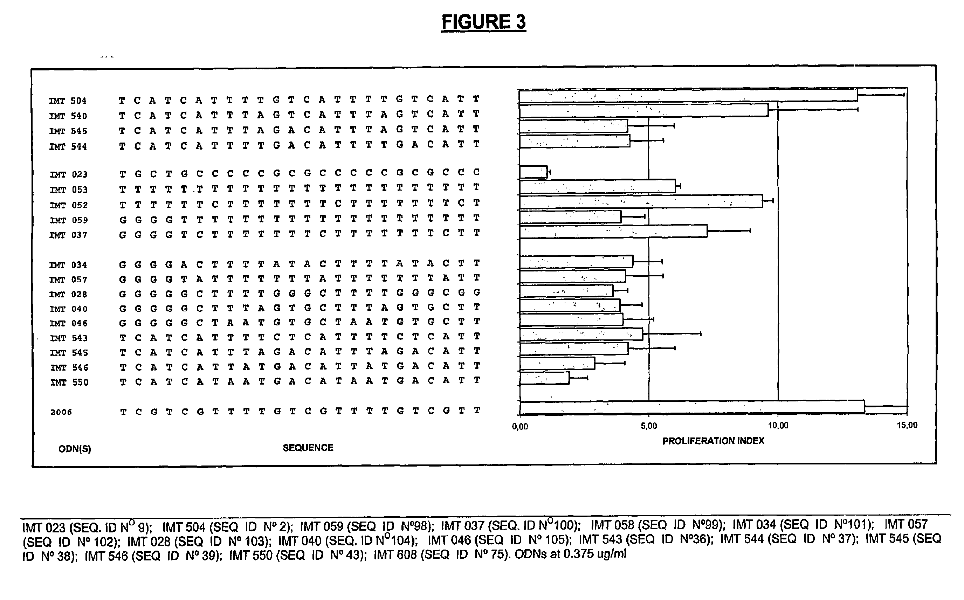

Fig. 3 is a graph plotting the proliferation index of PBMC incubated with the indicated phosphorothioate ODNs. Data represent the mean and standard deviation of five independent assays.

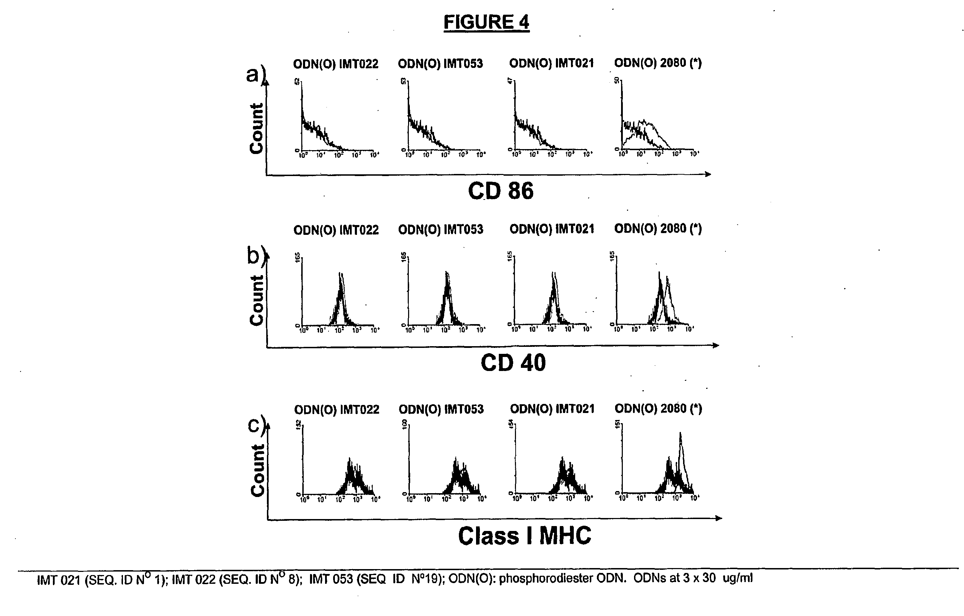

Fig. 4. shows the induction of CD86 in CD19+ (B) cells and CD69 in CD56 + (NK) cells by non-CpG ODNs claimed in WO 01/22972. Human PMBC were cultured for 48 hr with indicated *phosphodiester* ODNs or controls and then stained with fluorescent anti-CD19/anti-CD86 (a) or, anti-CD19/anti-CD40 (b) or, anti-CD19/anti-MHC I (c). Flow cytometric results are presented as histograms corresponding to cells in the double positive gate. Open histograms correspond to cells cultured in absence of ODN and shaded histograms to cells cultured in presence of ODN. ODN (O): phosphodiester ODN. ODN 2080, a CpG ODN of the following sequence: 5'-TCGTCGTTCCCCCCCCCCCC-3' was used as positive control.

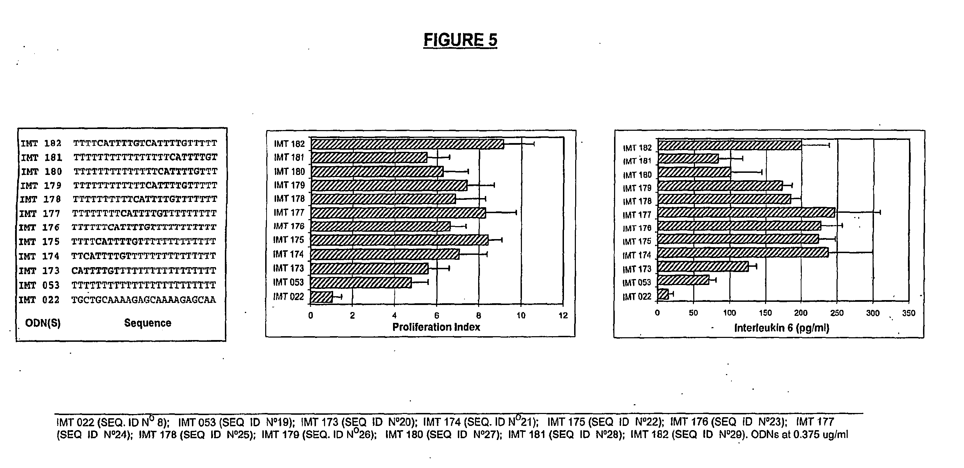

Fig. 5 shows the influence of the position of the immunostimulatory sequence motif X₁X₂X₃X₄X₅X₆X₇X₈ here disclosed on the immunostimulatory activity of non-CpG ODNs measured in PMBC proliferation assays. Data represent the mean and standard deviation of three independent assays performed in quadruplicate.

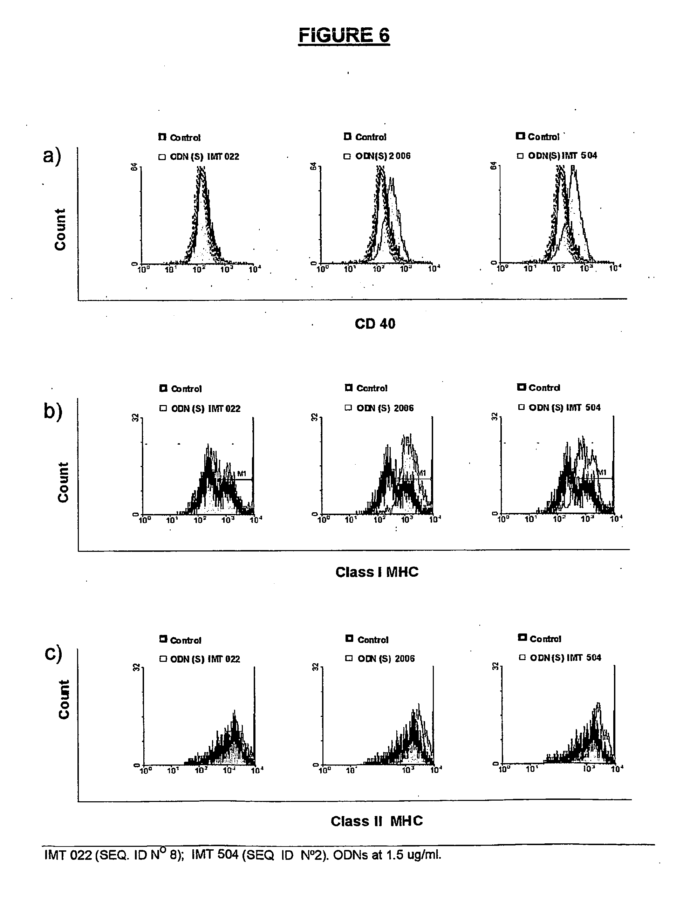

Fig. 6 shows the induction of CD40, MHC I and MHC II in CD19+ cells (B cells) by non-CpG-ODNs bearing the immunostimulatory sequence motif X₁X₂X₃X₄X₅X₆X₇X₈ here disclosed. Human PMBC were cultured for 24 hr with indicated ODNs and then stained with fluorescent anti-CD19/anti-CD40 (a) or, anti-CD19/ anti-MHC I (b) or, anti-CD19/ anti-MHC II (c). Flow-cytometric results are presented as histograms corresponding to CD19+ cells. Open histograms correspond to cells cultured in absence of ODN and shaded histograms to cells cultured in presence of ODN. ODN (S) means phosphorothioate ODN.

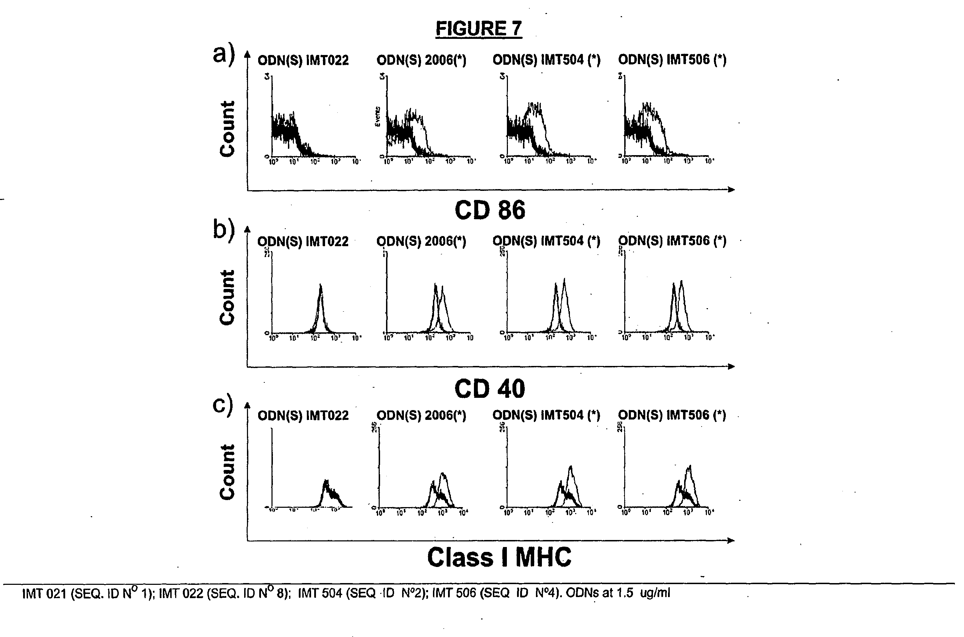

Fig. 7 shows the induction of CD86, CD40 and MHC I in purified B cells by non-CpG-ODNs bearing the immunostimulatory sequence motif X₁X₂X₃X₄X₅X₆X₇X₈ here disclosed. Human purified B cells were cultured for 24 hr with indicated ODNs and then stained with fluorescent anti-CD19/anti-CD86 (a) or, anti-CD19/anti-CD40 (b) or, anti-CD19/anti-MHC I (c). Flow-cytometric results are presented as histograms corresponding to CD19+ cells. Open histograms correspond to cells cultured in absence of ODN and shaded histograms to cells cultured in presence of ODN. ODN (S) means phosphorothioate ODN.

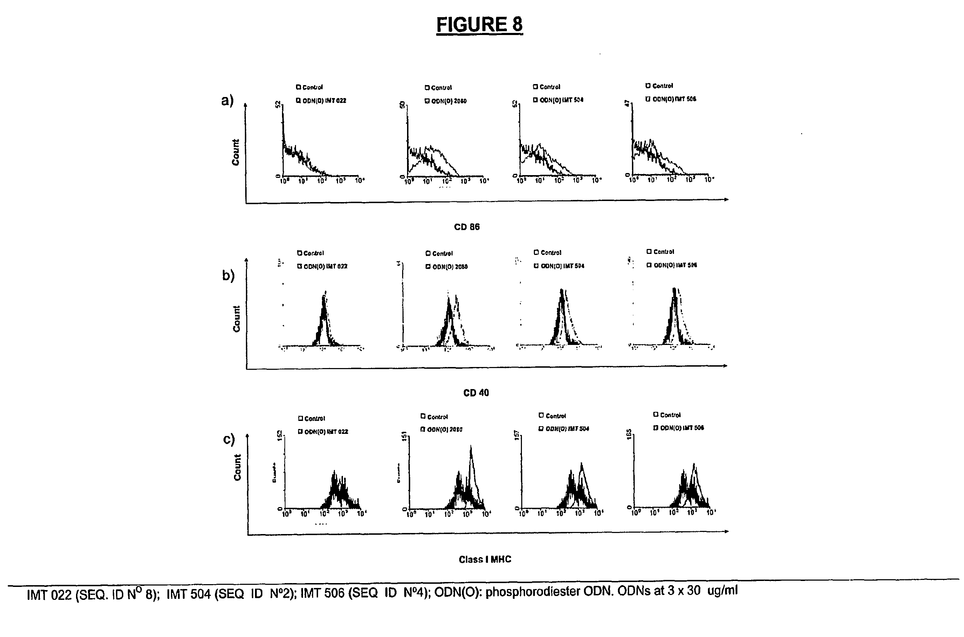

Fig. 8 shows induction of CD86, CD40 and MHC I in CD19+ cells (B cells) by phosphodiester non-CpG-ODNs bearing the immunostimulatory sequence motif X₁X₂X₃X₄X₅X₆X₇X₈ here disclosed. Human PMBC were cultured for 48 hr with indicated phosphodiester ODNs and then stained with fluorescent anti-CD19/anti-CD86 (a) or, anti-CD19/anti-CD40 (b) or, anti-CD19/anti-MHC I (c). Flow-cytometric results are presented as histograms corresponding to CD19+ cells. Open histograms correspond to cells cultured in absence of ODN and shaded histograms to cells cultured in presence of ODN. ODN (O) means phosphodiester ODN.

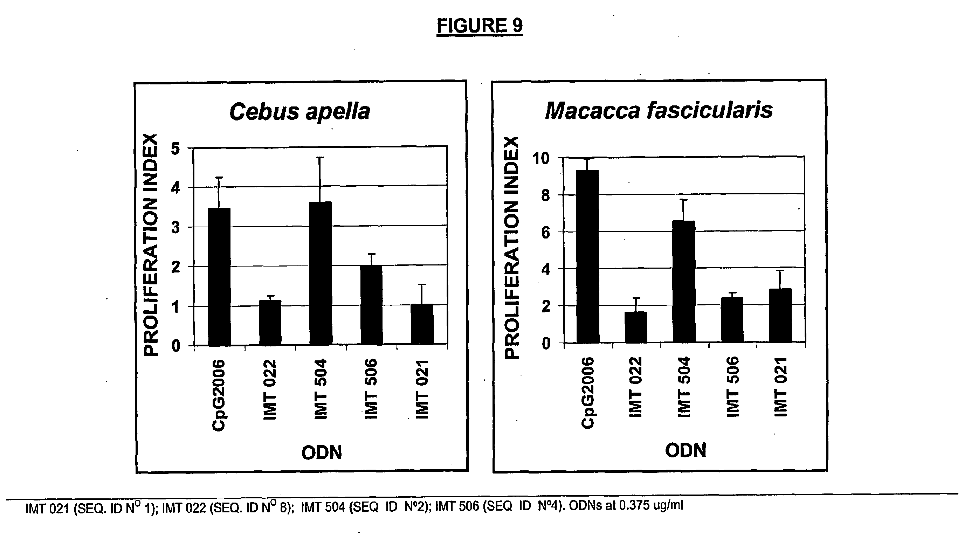

Fig. 9 shows stimulation of PMBC proliferation by non-CpG ODNs bearing the motif X₁X₂X₃X₄X₅X₆X₇X₈ here disclosed in non-human primates. *Cebus apella* or *Macacca fascicularis* PMBC were cultured for 72 hr with indicated ODNs (6µg/ml). Data represent the mean and standard deviation of four replicates.

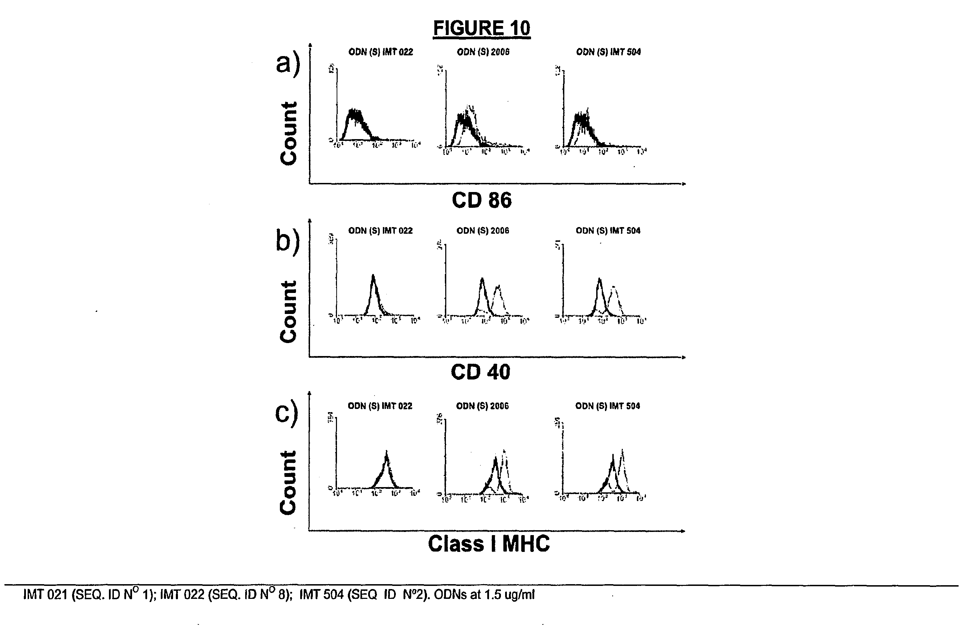

Fig. 10 shows the induction of CD86, CD40 and MHC I in CD 19+ (B cells) of a patient suffering B cell chronic lymphocytic leukemia (CLL) by non-CpG-ODNs bearing the motif X₁X₂X₃X₄X₅X₆X₇X₈ here disclosed. PMBC were cultured for 24hr with indicated ODNs and then stained with fluorescent anti-CD19/anti-CD86 (a) or, anti-CD19/anti-CD40 (b) or, anti-CD19/anti-MHC I (c). Flow-cytometric results are presented as histograms corresponding to CD19+ cells. Open histograms correspond to cells cultured in absence of ODN and shaded histograms to cells cultured in presence of ODN. ODN (S) means phosphorothioate ODN.

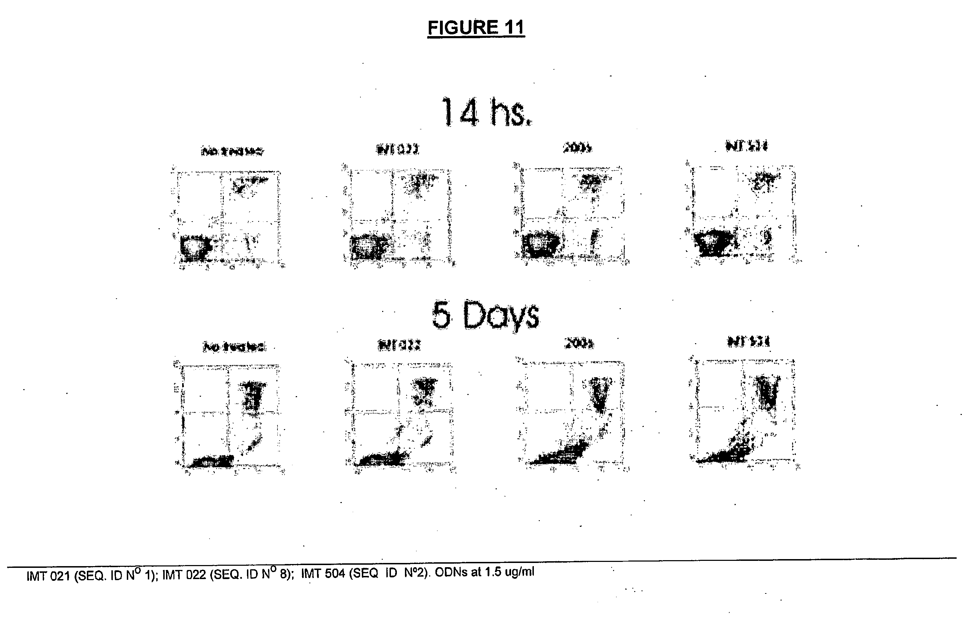

Fig. 11 shows the stimulation of malignant B cell apoptosis by non-CpG-ODNs bearing the motif X₁X₂X₃X₄X₅X₆X₇X₈ here disclosed. PMBC were cultured for 14 hr. or 5 days with indicated ODNs and then stained with propidium iodide and annexin V-FITC using the ANNEXIN V:FITC assay kit from Serotec (Raleigh, NC, USA). Flow-cytometric results are presented as a dot-plot of annexin V-FITC fluorescence versus propidium iodide (PI) fluorescence. This plot show three cell populations: a) low PI-low annexin that are viable cells, b) low PI-high annexin that are apoptotic cells and, c) high PI-high annexin that are secondary necrotic cells.

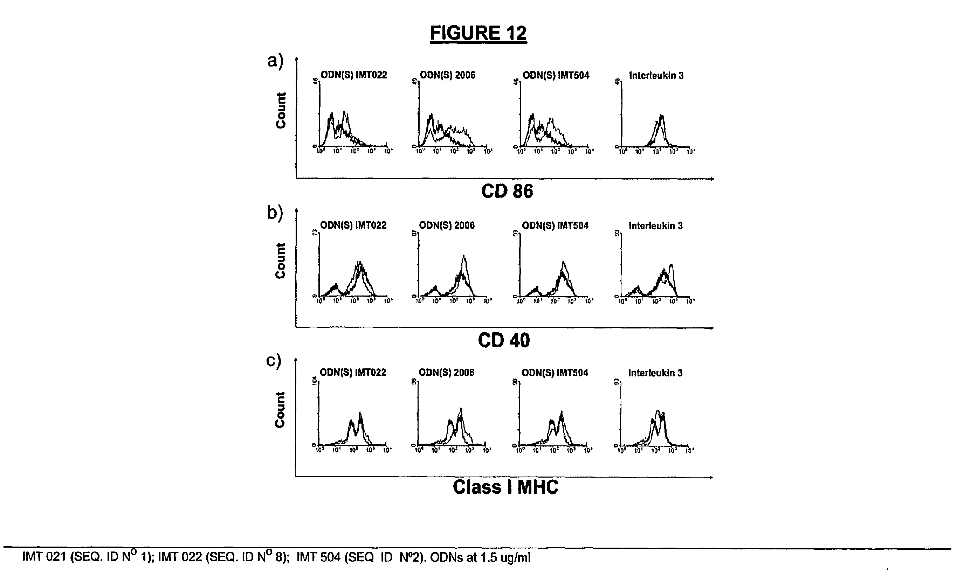

Fig. 12 shows the induction of CD86, CD40 and MHC I in purified plasmacytoid dendritic cells by non-CpG-ODNs bearing the motif X₁X₂X₃X₄X₅X₆X₇X₈ here disclosed. More than 95 % pure plasmacytoid dendritic cells were cultured for 24hr with indicated ODNs and then stained with fluorescent anti-CD4/anti-CD11c/anti-CD86 (a) or, anti-CD4/anti-CD11c /anti-CD40 (b) or, anti-CD4/anti-CD11c /anti-MHC I (c). Flow cytometric results are presented as histograms corresponding to CD4+CD11 c- cells. Open histograms correspond to cells cultured in absence of ODN and shaded histograms to cells cultured in presence of ODN. ODN (S) means phosphorothioate ODN.

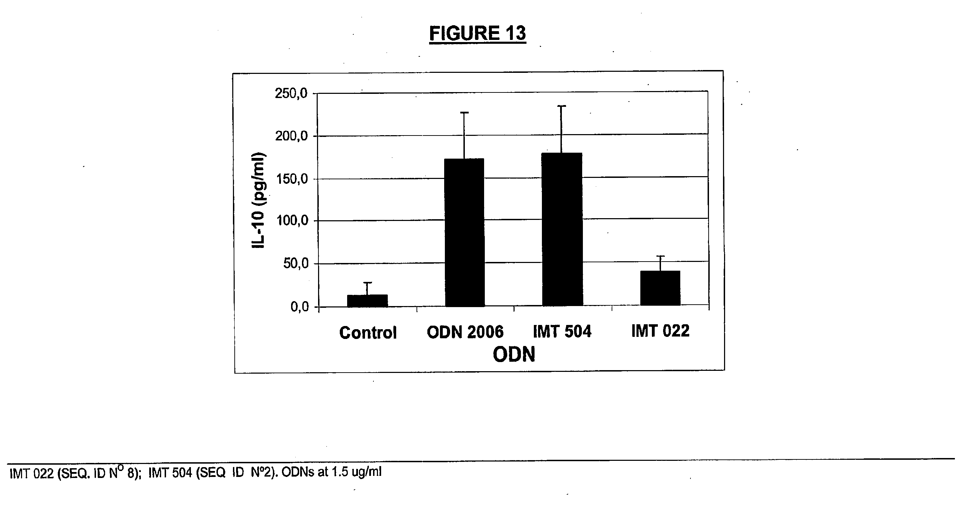

Fig. 13 is a graph plotting the IL-10 levels in supernatants of five independent human peripheral blood mononuclear leukocytes (PBMC) cultured 72 hr with the phosphorothioate non-CpG ODN IMT 504 (Seq. ID N° 2), the phosphorothioate CpG ODN 2006: 5' TCGTCGTTTTGTCGTTTTGTCGTT 3', or the phosphorothioate non-CpG ODN IMT 022 (SEQ ID NO: 175). Data represent the mean and standard deviation of five independent assays.

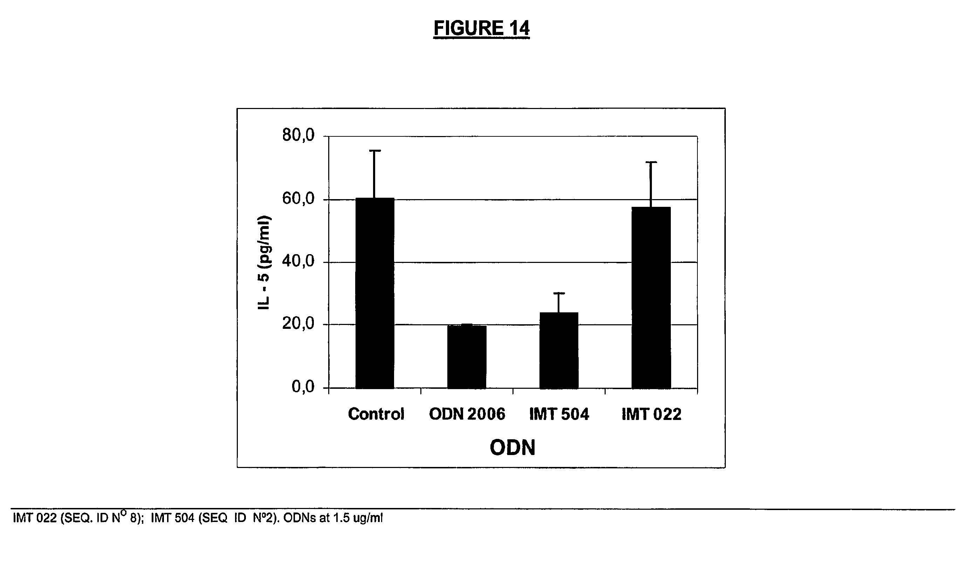

Fig. 14 is a graph plotting the IL-5 levels in supernatants of five independent human peripheral blood mononuclear leukocytes (PBMC) cultured 96 hs with the phosphorothioate non-CpG ODN IMT 504 (Seq. ID N° 2), the phosphorothioate CpG ODN 2006: 5' TCGTCGTTTTGTCGTTTTGTCGTT 3', or the phosphorothioate non-CpG ODN IMT 022 (SEQ ID NO: 175). Data represent the mean and standard deviation of five independent assays.

### DETAILED DESCRIPTION OF THE INVENTION

Definitions:
- An "allergy" refers to acquired hypersensitivity to a substance (allergen). Examples of allergies are eczema, allergic rhinitis, asthma and urticaria.
- An "immune system deficiency" refers to a disease in which the immune system is not functioning in normal capacity.
- As used herein, the term "oligonucleotide" or "oligo" shall mean multiple nucleotides (i.e. molecules comprising a sugar (e.g. ribose or deoxyribose) linked to a phosphate group and to an exchangeable organic base, which is either a substituted pyrimidine (e.g. cytosine (C), thymine (T) or uracil (U)) or a substituted purine (e.g. adenine (A) or guanine (G)). The term "oligonucleotide" as used herein refers to both oligoribonucleotides (ORNs) and oligodeoxyribonucleotides (ODNs). The term "oligonucleotide" shall also include oligonucleosides (i.e. an oligonucleotide minus the phosphate) and any other organic base containing polymer. Oligonucleotides can be obtained from existing nucleic acid sources (e.g. genomic or cDNA), but are preferably synthetic (e.g. produced by oligonucleotide synthesis).

- An "oligonucleotide" refers to multiple nucleotides linked by phosphodiester bonds.
- An "immunostimulatory oligonucleotide" refers to an oligonucleotide which stimulates (i.e. has a mitogenic effect on, or induces or increases or decreases cytokine expression by) a cell of the immune system (i.e.: a lymphocyte, a macrophage) in a statistically significant manner.
- A "Strong immunostimulatory oligonucleotide" refers to an oligonucleotide which stimulates "in vitro" cells of the immune system of an animal of the order Primate with an activity of at least 60% of the immunostimulatory activity of the well known CpG ODN 2006, under the same experimental conditions and using identical chemical backbone (i.e. phosphorotioate backbone or phosphodiester backbone).
- A "CpG" refers to a cytosine-guanine dinucleotide.
- A "CpG oligonucleotide" refers to an oligonucleotide which stimulates a cell of the immune system and its immunostimulatory activity critically depends on the presence of at least one CpG in its sequence.
- A "non-CpG oligonucleotide" refers to an oligonucleotide which stimulates a cell of the immune system and its immunostimulatory activity does not critically depends on the presence of a CpG in its sequence.
- A "subject" refers to an animal of the order Primate, including humans.
- As used herein, the term "treating" refers to a process by which the symptoms of a disease, and more particularly, infectious diseases, tumoral diseases or immunological disorders are ameliorated or completely eliminated.
- As used herein, the term "preventing" refers to a process by which a disease, and more particularly infectious diseases or tumoral diseases or immunological disorders are obstructed or delayed.
- In a preferred embodiment, the immunostimulatory oligonucleotides of the invention are advantageously modified into stabilized oligonucleotides. Such stabilized immunostimulatory oligonucleotide may be particularly useful to obtain a prolonged immunostimulation. As used herein, a "stabilized oligonucleotide" refers to an oligonucleotide that is relatively resistant to in vivo degradation (e.g. via an exo- or endo-nuclease). Preferred stabilized oligonucleotides of the present invention comprise a phosphate backbone modification. More particularly, the phosphate backbone modification is preferably a 5' inter-nucleotide linkage modification, for instance, at the first two nucleotides of the 5' end of the oligonucleotide of the invention. Furthermore, the phosphate backbone modification may be a 3' inter- nucleotide linkage modification. In such a case, the modification may occur, for instance, at the last two nucleotides of the 3' end of the oligonucleotide of the invention. Even more preferably, the immunostimulatory oligonucleotide of the invention may be stably modified so as to comprise a phosphorothioate-linked nucleotide (i.e. at least one of the phosphate oxygens is replaced by sulfur). In the most preferred embodiment, most if not all the nucleotides of the immunostimulatory oligonucleotides of the invention comprise a phosphorothioate-linked nucleotide.

Other stabilized oligonucleotides may alternatively include: nonionic DNA analogs, such as alkyl- and aryl- phosphonates (in which the charged phosphonate oxygen is replaced by an alkyl or aryl group), phosphodiester and alkylphosphotriesters, in which the charged oxygen moiety is alkylated. Oligonucleotides which contain a diol, such as tetraethyleneglycol or hexaethyleneglycol, at either or both termini have also been shown to be substantially resistant to nuclease degradation.

The present invention provides methods to augment the immune response of animals of the order *Primate,* including humans, adding one or more of the oligonucleotides of this invention to vaccines, or performing a treatment based on the administration of one or more of the oligonucleotides of this invention to a person with a tumoral disease or an immunological disorder, or contacting "ex vivo" white blood cells obtained from a person with a tumoral disease or an immunological disorder with one or more of the oligonucleotides of this invention and readministering these activated white blood cells to the same person.

Vaccine compositions containing one or more of the oligonucleotides of this invention can present antigens directly (i.e. in the form of a defined protein or polysaccharide) or as a part of a complex biological entity (i.e. complete viruses; complete bacterial cells; bacterial membranes or artificial conjugates like polysaccharide-protein conjugates). These antigens can be combined in multiple vaccines.

A vaccine composition including at least one antigen is formulated to include one or more of the oligonucleotides of this invention.

For example the antigen can be *Moraxella catharralis* killed cells or subcellular fractions of these cells or *Hepatitis B virus* surface antigen, either natural or produced by means of the DNA recombinant technology.

One or more of the oligonucleotides of this invention may be formulated alone or together with one or more antigens in a pharmaceutical composition, which may also include carriers, thickeners, diluents, buffers, preservatives, surface active agents, anti-microbial agents, anti-inflammatory agents, anesthetics and the like.

The formulation can be liquid or lyophilized. The pharmaceutical composition may be administered in a number of ways depending of whether local or systemic treatment is desired, and on the area to be treated. Administration may be done topically, orally, by inhalation or parenterally. Formulation for topical administration may include ointments, lotions, creams, gels, drops, suppositories, sprays, liquids and powders. Pharmaceutical carriers, aqueous, powder or oily bases, thickeners and the like may be necessary or desirable. Formulations for oral administration include powders or granules, suspensions or solutions in water or non-aqueous media, capsules, tablets and the like. Thickeners, flavorings, diluents, emulsifiers and the like may be necessary or desirable. Formulations for parenteral administration include sterile aqueous solutions, which may also contain buffers, diluents and other additives. A vaccine containing one or more antigens and one or more of the oligonucleotides of this invention can be formulated and used for prophylactic or therapeutic purposes.

Common antigens used in viral prophylactic vaccines are from *Hepatitis B virus, Hepatitis A virus* and *Influenza virus.* Common antigens used in bacterial prophylactic vaccines are from Streptococcus *pneumoniae, Haemophilus influenzae, Moraxella catharralis, Klebsiella pneumoniae and Mycobacterium bovis (BCG).* Common antigens used in therapeutic vaccines are from *Papilloma virus, HIV virus* and melanoma cells.

A further refinement of a vaccine formulation is to incorporate one or more of the oligonucleotides of this invention as adjuvant/s and the antigen/s into a delivery vehicle to provide for delayed release of the active compounds of the vaccine over time. This can be accomplished by various means known in the art. Examples of these means are encapsulation into Poly (lactide-coglicolide) micro particles (Kersten, G.F.A. and Gander, B. 1996. Biodegradable Micro Spheres as vehicles for antigens, in: S.H.E. Kaufmann, ed. Concepts in Vaccine Development. Walter de Gruyter. Berlin-New York), liposomes (Gregoriadis, G. et al. 2000. Liposomes as Immunological Adjuvants and Vaccine Carriers, in: S.H.E. Kaufmann, ed. Concepts in Vaccine Development. Walter de Gruyter. Berlin-New York) and poly (methyl methacrylate) nanoparticles (Kreuter, J. 2000. Poly (Methyl Methacrylate) nanoparticles as vaccine adjuvants, in: S.H.E. Kaufmann, ed. Concepts in Vaccine Development. Walter de Gruyter. Berlin-New York).

Another refinement of the vaccine formulation is to conjugate the antigen/s and one or more of the oligonucleotides of this invention, by chemical means (Mier W, Eritja R, Mohammed A, Haberkorn U, Eisenhut M.2000. Preparation and evaluation of tumor-targeting peptide-oligonucleotide conjugates. Bioconjug. Chem. 11:855).

Many vaccine formulations are known in the art and can be used by substituting one or more of the oligonucleotides of this invention for the adjuvant previously known or by simply adding one or more of the oligonucleotides of this invention to the original formulation.

Based on their immunostimulatory properties, one or more of the oligonucleotides of this invention can also be administered to a subject in vivo to treat a tumoral disease or an immune system disorder.

Examples of common tumoral diseases are: Chronic Myelogenous Leukemia, Melanoma, Kaposi's Sarcoma, Multiple Myeloma, Renal Cell Carcinoma, Bladder Cancer, Lung Cancer, Skin Cancer, Breast Cancer, Colon Cancer and Uterus Cancer. Examples of common immunological disorders are: Allergy, Severe Combined Immunodeficiency, Chronic Granulomatous disease, and Acquired Immunodeficiency Disease.

The pharmaceutical composition for these treatments may include one or more of the oligonucleotides of this invention together with carriers, thickeners, diluents, buffers, preservatives, surface active agents, anti-microbial agents, anti-inflammatory agents, anesthetics and the like. The formulation can be liquid or lyophilized.

The pharmaceutical composition may be administered in a number of ways depending on whether local or systemic treatment is desired, and on the area to be treated. Administration may be done topically, orally, by inhalation or parenterally. Formulation for topical administration may include ointments, lotions, creams, gels, drops, suppositories, sprays, liquids and powders. Pharmaceutical carriers, aqueous, powder or oily bases, thickeners and the like may be necessary or desirable. Formulations for oral administration include powders or granules, suspensions or solutions in water or non-aqueous media, capsules, tablets and the like. Thickeners, flavorings, diluents, emulsifiers and the like may be necessary or desirable. Formulations for parenteral administration include sterile aqueous solutions, which may also contain buffers, diluents and other additives. Alternatively, one or more of the oligonucleotides of this invention can be contacted "*ex vivo"* with immunocompetent cells (i.e. B cells or plasmacytoid dendritic cells) obtained from a subject having a tumoral disease or an immune system deficiency and activated cells can then be reintroduced in the subject.

As it will be apparent from the following examples the preferred oligonucleotides are those which stimulate "in vitro" cells of the immune system of an animal of the order Primate with an activity of at least 60% of the immunostimulatory activity of the well known CpG ODN 2006, under the same experimental conditions and using identical chemical backbone (i.e the strong immunostimulatory oligonucleotides).

Among these are preferred those having up to 100 nucleotides, more preferably up to 40 nucleotides, and comprising the immunostimulatory oligonucleotide IMT 504, namely TCATCATTTTGTCATTTTGTCATT (SEQ I D N°2), which represents the main object of the present invention.

### EXAMPLE 1

### Materials and Methods

The following materials and methods were generally used throughout the examples.

### 1) Oligonucleotides

Oligonucleotides having phosphorothioate internucleotide linkages were purchased, purified by high-pressure liquid chromatography (HPLC), from Operon Technologies (Alameda, California) or Annovis (Aston, Pennsylvania) or Oligos Etc (Bethel, Maine). ODNs were suspended in depyrogenated water, assayed for LPS contamination using the Limulus test and kept at -20 °C until used. Purity was assessed by HPLC and PAGE assays. ODN preparations were used if LPS levels were undetectable.

### 2) Antibodies

Antibodies used in assays were purchased from Serotec (Raleigh, NC).

### 3) Peripheral blood mononuclear leukocytes (PBMC)

Blood was obtained by venipuncture from healthy donors using heparin as anticoagulant. PMBC were isolated by Ficoll-Hypaque (Sigma Diagnostics Inc., St. Louis, MO) density gradient centrifugation. Briefly, blood samples diluted 1:2 in RPMI-1640 medium (PAA laboratories GmbH, Linz, Austria) supplemented with 2.0 mM L-glutamine and 50.0 µg/ml gentamicin and 20 mM HEPES were centrifuged at 1000 × g for 40 minutes at 20 °C. PMBC were isolated, washed and suspended in medium supplemented with 10 % fetal calf serum.

### 4) Purification of cells

B lymphocytes and plasmacytoid dendritic cells were purified from human PMBC by positive selection using the MACS magnetic cell separation systems (Miltenyi Biotec, Germany).

### 5) Cell Proliferation assays

Blood was obtained by venipuncture from healthy donors using heparin as anticoagulant. PMBC were isolated by Ficoll-Hypaque (Sigma Diagnostics Inc., St. Louis, MO) density gradient centrifugation. Briefly, blood samples diluted 1:2 in RPMI-1640 medium (PAA laboratories GmbH, Linz, Austria) supplemented with 2.0 mM L-glutamine and 50.0 µg/ml gentamicin and 20 mM HEPES were centrifuged at 1000 × g for 40 minutes at 20 °C. PMBC were isolated, washed and suspended in medium supplemented with 10 % fetal calf serum.

PBMC were cultured in RPMI-1640 media supplemented with 10% (v/v) heat inactivated fetal calf serum (FCS), 2.0 mM L-glutamine and 50.0 µg/ml gentamicin. 1×10⁵ cells/well were incubated in 96 well microtiter plates (NUNC, Denmark) at 37 °C in a 5% CO₂ humidified atmosphere for 72 hr. PBMC were stimulated with ODNs at 0.375 µg/ml unless otherwise stated. Eighteen hours prior to cell harvest, 1 µCi of ³H-Thymidine (Amersham, specific activity: 25 Ci/mmol) was added to each well. Cells were harvested onto glass-fiber filters and ³H incorporation measured by scintillation counting. Standard deviations of quadruplicate wells were <10%.

### 6) IL6 Assay

PBMC (3×10⁵/well) were cultured as described above with ODNs (6 µg/ml) for 24 hr. After this, supernatants were collected and IL6 levels measured by ELISA. Briefly, 96 well micro titer plates (NUNC, Denmark) were coated with anti-IL6 antibodies and blocked with RPMI 1640 media supplemented with 10% (v/v) heat inactivated FCS. IL6 was detected colorimetrically using biotin-labeled antibodies followed by peroxidase-conjugated strepto-avidin and then peroxidase-specific colorimetric substrate. Standard curves were generated using known amounts of recombinant IL6. The detection limit of these assays was 30 µg/ml. All assays were performed in duplicate.

### 7) IL-10 Assay

PBMC (3×10⁵/well) were cultured as described above with ODNs (1.5 µg/ml) for 72 hr. After this, supernatants were collected and IL-10 levels measured by ELISA. Briefly, 96 well micro titer plates (NUNC, Denmark) were coated with anti-IL-10 antibodies and blocked with RPMI 1640 media supplemented with 10% (v/v) heat inactivated FCS. IL-10 was detected colorimetrically using biotin-labeled antibodies followed by peroxidase-conjugated strepto-avidin and then peroxidase-specific colorimetric substrate. Standard curves were generated using known amounts of recombinant IL-10. The detection limit of these assays was 20 pg/ml. All assays were performed in duplicate.

### 8) IL-5 Assay

PBMC (3×10⁵/well) were cultured as described above with ODNs (1.5 µg/ml) and IL-4 (5 ng/ml) for 72 hr. After this, supernatants were collected and IL-5 levels measured by ELISA. Briefly, 96 well micro titer plates (NUNC, Denmark) were coated with anti-IL-5 antibodies and blocked with RPMI 1640 media supplemented with 10% (v/v) heat inactivated FCS. IL-5 was detected colorimetrically using biotin-labeled antibodies followed by peroxidase-conjugated strepto-avidin and then peroxidase-specific colorimetric substrate. Standard curves were generated using known amounts of recombinant IL-5. The detection limit of these assays was 40 pg/ml. All assays were performed in duplicate.

### 9) IgM Secretion Assay

PBMC (3×10⁵/well) were cultured as described above with ODNs (1.5 µg/ml) for 72 hr. After this, supernatants were collected and IgM assayed by ELISA. Briefly, 96 microtiter plates (NUNC, Denmark) were coated with anti-IgM antibodies and blocked with RPMI 1640 media. IgM was detected colorimetrically using peroxidase-labeled antibodies followed by peroxidase-specific colorimetric substrate. Standard curves were generated using known amounts of purified IgM. The detection limit of these assays was 50 ng/ml. All assays were performed in duplicate.

### 10) Flow cytometry

Staining of surface antigens was performed as described (J. Fló and E. Massouh. Age-related changes of native and memory CD4 rat lymphocyte subsets in mucosal and systemic lymphoid organs. Developmental and comparative Immunology 21: 443-453, 1997). Anti CD19 (Clone LT19), CD86 (Clone BU63), CD40 (clone LOB 7/6), CD4 (clone S 3.5), MHC class I (Clone W6/32), MHC class II (Clone WR 18), anti CD4 (Clone S3.5) and anti CD11c (Clone BU15) antibodies were purchased from Serotec (Raleigh, NC, USA). Apoptosis was estimated using the ANNEXIN V:FITC assay kit from Serotec (Raleigh, NC, USA).

Flow cytometric data of 10,000 cells/sample were acquired on a FACScan (Becton Dickinson Immunocytometry Systems, San Jose, CA). Data were analyzed using the computer program Win MDI, 2.8 , Interface Flow Cytometry Application

(Joseph Trotter Copyright 1993-1998).

### 11) Immunization of monkeys against hepatitis B surface antigen (HBsAg) and evaluation of the humoral response

Twelve monkeys of the species *Cebus Apella* (2.5-3.5 Kg) were immunized with a pediatric dose of AgB (Pablo Cassará, Buenos Aires, Argentina) containing 10 µg of HBsAg adsorbed to alumina (25 mg of Al ³⁺/ mg of HBsAg). This was administered alone (n= 3, 1 female, 2 males) or combined with indicated phosphorothioate ODN (150 µg/dose) (n=3, 1 female, 2 males). All vaccines were administered intramuscularly (i.m.) in the quadriceps muscle in a total volume of 1 ml. Monkeys were kept in the animal facility of the CEMIC (Centro Médico de Investigaciones Clínicas), Buenos Aires, Argentina. Animals were monitored daily by specialists and weighed once a week. Plasma was recovered by intravenous (i.v.) puncture before and at various times after immunization and immediately assayed for antibodies using the commercial kit AUSAB (Abbot Laboratories, Illinois, USA). Titers are expressed in milliinternational units per ml.

### EXAMPLE 2

### Selection of oligonucleotide sequences

WO 96/02555 and US 6,239,116 patents teach that to be immunostimulatory, oligonucleotides require sequences containing unmethylated CpG motifs (WO 96/02555, col. 13 lines 19-20 and US 6,239,116, col. 6 lines 1-3). EP 0 468 520 teaches that to be immunostimulatory, oligonucleotides require a palindromic sequence of at least 6 nucleotides long to be satisfactory (EP 0 468 520, col. 11, lines 34-37). Therefore, several non-CpG oligonucleotides, without palindromic sequences of at least 6 nucleotides long were proved using proliferation assays, cell differentiation assays, cytokine IL6 secretion assays and IgM secretion assays performed on human peripheral blood mononuclear leukocytes (PBMC). As a positive control, the CpG oligonucleotide 2006 of composition :
5' TCGTCGTTTTGTCGTTTTGTCGTT 3'
and phosphorothioate bonds described by Hartman and Krieg (Hartmann, G., Krieg, A.M. 2000. Mechanism and function of a newly identified CpG DNA motif in human primary B cells. J. Immunol. 164:944.) was used. As a background control, either the phosphorothioate oligonucleotide IMT 023 (SEQ ID N°9) or IMT 022 (SEQ ID N°8) with very low activity on human cells was used.

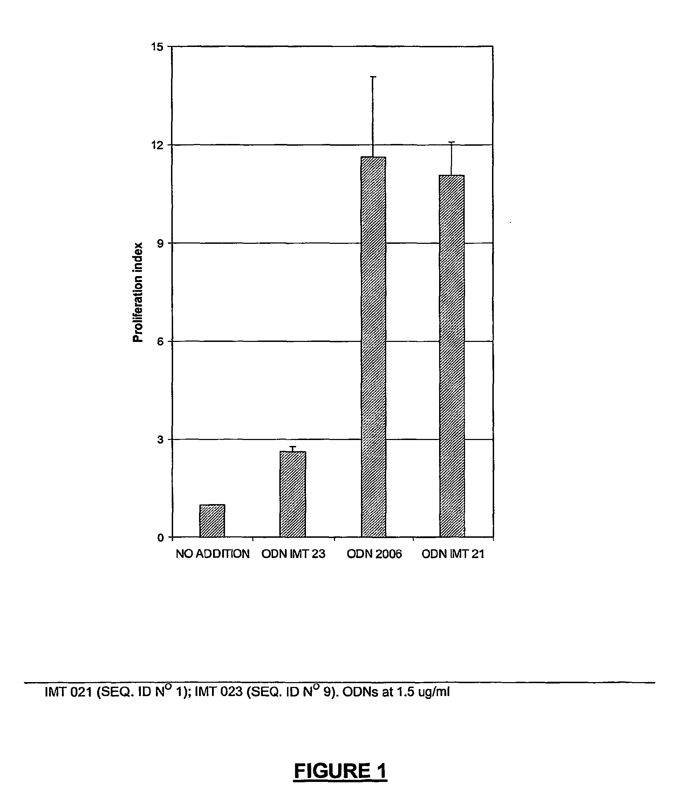

Also as a "presumably" negative control an oligonucleotide with the same composition of the oligonucleotide 2006 but in which all the CpG dinucleotides have been replaced by GpC dinucleotides:
5' TGCTGCTTTTGTGCTTTTGTGCTT 3'
was used. This oligonucleotide was named ODN IMT 021 (SEQ ID N° 1). Fig. 1 shows a proliferation assay performed with the above described oligonucleotides.

It was found, surprisingly, that the non CpG oligonucleotide IMT 021 was as active as the 2006 CpG oligonucleotide in proliferation assays (Fig. 1 and Table 1) if used at 1.5 µg/ml and approximately 40-60% active if used at 0.375 µg /ml.

**TABLE 1**

| Induction of peripheral white blood cell proliferation by Phosphorothioate | | | | | | | |
|---|---|---|---|---|---|---|---|
| CpG (2006) and non-CpG oligonucleotides (IMT 021) | | | | | | | |
| | | | | | | | |
| *ODN (S)* | *SEQUENCE (5'-3)* | *PROLIFERATION INDEX* | | | | | |
| | | *(ODN at 1.5 µg*/*ml)* | | | *(ODN at 0.375* µ*g*/*ml)* | | |
| | | *Avg.* | *N* | *SD* | *Avg.* | *N* | *SD* |
| 2006 | TCGTCGTTTTGTCGTTTTGTCGTT | 17.95 | 4 | 2.39 | 13.83 | 14 | 1.55 |
| IMT 021 | TGCTGCTTTTGTGCTTTTGTGCTT | 17.41 | 5 | 2.74 | 8.02 | 15 | 1.21 |

| | | | | | | | |
|---|---|---|---|---|---|---|---|
| IMT 021 (SEQ. ID N° 1) *Avg.: Average; N: Number of Data; SD: Standard Deviation;* ODN(S): phosphorothioate ODN | | | | | | | |

Immunostimulation was also evaluated by Flow Cytometry using the CD 19 general marker for human B cells and the CD 86 activation marker for human B cells.

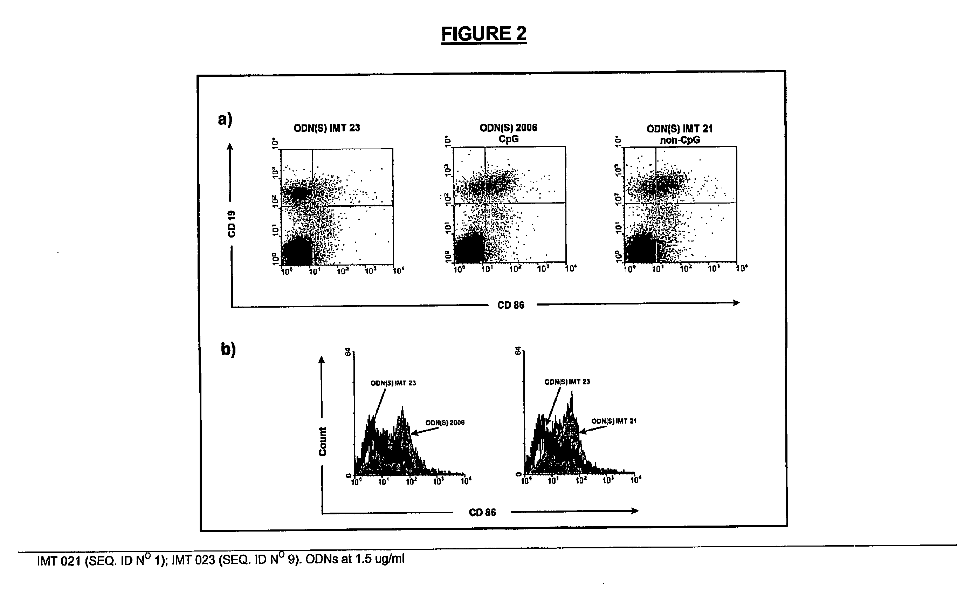

Fig. 2 shows the activation of human B cells incubated with the non CpG ODN (S) IMT 021 as compared to the activation induced by incubation with the CpG ODN (S) 2006. As can be observed, both ODNs show similar activation patterns as compared with the ODN(S) IMT 023 background control when used at 1.5 ug/ml.

IL6 secretion was also evaluated in supernatants of PBMC incubated with the CpG ODN (S) 2006 and the non CpG ODN (S) IMT 021 (Table 2).

**TABLE 2**

| Induction of human IL-6 secretion bv phosphorothioate | | | | |
|---|---|---|---|---|
| CpG and non-CpG oligonucleotides 2006 and IMT 021 | | | | |
| | | | | |
| *ODN (S)* | *IL-6 (pg*/*ml)* | | | |
| | expt. 1 | expt. 2 | expt. 3 | expt. 4 |
| cells alone | 0 | 0 | 0 | 0 |
| 2006 | 568 | 159 | 597 | 374 |
| IMT 021 | 348 | 384 | 836 | 596 |

| | | | | |
|---|---|---|---|---|
| IMT 021 (SEQ. ID N° 1). ODNs at 1.5 ug/ml | | | | |

Results of this assay also indicate that the non-CpG ODN (S) IMT 021 is an effective immunostimulant when used at 1.5 ug/ml. Therefore, other non-CpG sequence variants of the 2006 oligonucleotide were investigated. Table 3 shows the results for six of these variants in which, the Cs or Gs of all the CpGs of this ODN were replaced by other nucleotides. As can be observed, the Gs of the CpGs in the ODN(S) 2006 are not necessary for B cell proliferation or IL6 secretion. However, modification of the Cs of the CpGs is detrimental if the replacement is for As or Gs but not if it is for Ts. These results clearly indicate that stimulation of the B cell proliferation and IL6 secretion by the ODN(S) 2006 *is not at all associated with integrity of the CpG group.*

**TABLE 3**

| Induction of peripheral white blood cell proliferation and IL6 secretion by non- | | | | | | | |
|---|---|---|---|---|---|---|---|
| CpG variants of the ODN(S) 2006 oligonucleotide | | | | | | | |
| | | | | | | | |
| *ODN (S)* | *SEQUENCE* (5'-3') | *PROLIFERATION INDEX* | | | *IL-6 (pg*/*ml)* | | |
| | | *(ODN at 0.375* µ*g*/*ml)* | | | (ODN at 6.0 µg/ml) | | |
| | | *Avg.* | *N* | *SD* | *Avg.* | *N* | *SD* |
| 20 | | | | | | | |
| 2006 | TCGTCGTTTTGTCGTTTTGTCGTT | 13.83 | 14 | 1.55 | 215.8 | 4 | |
| 83.4 | | | | | | | |
| IMT 504 | TC**A**TC**A**TTTTGTC**A**TTTTGTC**A**TT | | 13.08 | 2 | 3.80 | 285.2 | 2 |
| 39.5 | | | | | | | |
| IMT 505 | TC**C**TC**C**TTTTGTC**C**TTTTGTC**C**TT | | 12.98 | 2 | 4.73 | 218.9 | 2 |
| 3.1 | | | | | | | |
| IMT 506 | TC**T**TC**T**TTTTGTC**T**TTTTGTC**T**TT | | 11.66 | 4 | 2.77 | 228.9 | 4 |
| | 161.0 | | | | | | |
| 10 | | | | | | | |
| IMT 501 | T**A**GT**A**GTTTTGT**A**GTTTTGT**A**GTT | | 5.42 | 2 | 2.03 | 66.8 | 2 |
| 54.0 | | | | | | | |
| IMT 502 | T**G**GT**G**GTTTTGT**G**GTTTTGT**G**GTT | | 7.16 | 2 | 3.24 | 89.7 | 2 |
| 95.4 | | | | | | | |
| IMT 503 | T**T**GT**T**GTTT**T**GT**T**GTTTTGT**T**GTT | | 9.87 | 2 | 1.90 | 334.9 | 2 |
| | 215.5 | | | | | | |

| | | | | | | | |
|---|---|---|---|---|---|---|---|
| IMT 504 (SEQ ID N°2); IMT 505 (SEQ ID N°3); IMT 506 (SEQ ID N°4); IMT 501 (SEQ ID N°5); IMT 502 (SEQ ID N°6); IMT 503 (SEQ ID N°7) | | | | | | | |

### EXAMPLE 3

### Effect of structure modification on the immunostimulatory activity of non-CpG oligonucleotides: definition of the active motif.

In order to study the influence of the primary structure on the immunostimulatory activity of the non-CpG ODNs, several variants of the 2006 and IMT 021 oligonucleotides were synthesized. Table 4 shows the primary structure of some of the IMT 021 variants and the results of a proliferation and IL-6 assays performed in order to evaluate its immunostimulatory activity.

The ODN(S) IMT 021 contains T in positions 7,8,9,10,12,15,16,17,18,20,23 and 24. Replacement of these Ts with As (ODN IMT 022) or Cs (ODN IMT 023) results in a very significant loss of activity (about 76% and 75% respectively) in the proliferation assay and also in the IL-6 secretion assay (84% and 88% respectively). These results indicate that some or all the Ts in positions 7,8,9,10,12,15,16,17,18,20,23 and 24 are critical for the ODN IMT 021 immunostimulatory activity.

**TABLE 4**

| Induction of peripheral white blood cell proliferation and IL-6 secretion by | | | | | | | |
|---|---|---|---|---|---|---|---|
| Phosphorothioate non-CpG oligonucleotides derived from the | | | | | | | |
| inmunostimulatory IMT 021 oligonucleotide | | | | | | | |
| | | | | | | | |
| *ODN (S)* | *SEQUENCE (5'-3')* | *PROLIFERATION INDEX* | | | *IL-6 (pg*/*ml)* | | |
| | | (*ODN at 0.375 µq*/*ml*) | | | (*ODN at 6.0* µ*g*/*ml*) | | |
| | | *Avg.* | *N* | *SD* | *Avg.* | *N* | *SD* |
| IMT 021 | TGCTGCTTTTGTGCTTTTGTGCTT | 8.02 | 15 | 1.21 | 181.3 | 4 | 49.9 |
| IMT 022 0.0 | TGCTGC**AAAA**G**A**GC**AAAA**G**A**GC**AA** | 1.24 | 2 | 0.30 | 0.0 | 2 | |
| IMT 023 0.0 | TGCTGC**CCCC**G**C**GC**CCCC**GCGC**CC** | 1.07 | 4 | 0.12 | 0.0 | 3 | |

| | | | | | | | |
|---|---|---|---|---|---|---|---|
| IMT 021 (SEQ. ID N° 1); IMT 022 (SEQ. ID N° 8); IMT 023 (SEQ. ID N° 9) | | | | | | | |

In order to investigate the composition of the active center (motif) of the non-CpG oligonucleotides, several ODNs were assayed at 0.375 ug/ml(Fig. 3). It was found that:
a) The presence of TG groups in an oligonucleotide is not at all sufficient (in disagreement with WO 01-22972) for a strong immunostimulation. For example, ODNs IMT 028, IMT 046, IMT 546, IMT 547 and IMT 550, all of which have two TGs, have very poor activity. On the other hand, ODN IMT 543 that does not possess a TG dinucleotide has the same (low) activity as ODN IMT544, which is an ODN with the same general composition, but which contains two TG dinucleotides.
b) The presence of the tetranucleotide TTTT in an oligonucleotide is not at all sufficient (in disagreement with WO 01-22972) for a strong immunostimulation. For example, ODNs IMT 034, IMT 057, IMT 028, IMT 543 and IMT 544, which have two TTTT motifs, have very poor activity.
c) The presence of more than 25% of Ts is not at all sufficient (in disagreement with WO 01-22972) for a strong immunostimulation. For example, ODNs IMT 034(50% T), IMT 057 (71% T), IMT 028 (33% T), IMT 543(58% T), IMT 544 (50% T), IMT 059 (83% T), IMT 040 (42% T), IMT 046 (33% T), IMT 545 (42% T), IMT 550 (33% T) and IMT 546(42% T), all of which have more than 25% of T, have very poor activity.
d) The presence of more than 25% of Ts plus the presence of TG dinucleotides, is not at all sufficient (in disagreement with WO 01-22972) for strong stimulation. For example, ODN IMT 028, IMT 046, IMT 544 and IMT 546, which have more than 25% of Ts and two TGs, have very poor activity.
e) The presence of more than 25% of Ts plus the presence of the tetranucleotide TTTT, is not at all sufficient (in disagreement with WO 01-22972) for strong stimulation. For example, ODN IMT 059, IMT 034, IMT 057, IMT 028, IMT 543 and IMT 544, which have more than 25% of Ts and two TTTT, have very poor activity.
f) The presence of TG dinucleotides plus the presence of the tetranucleotide TTTT is not at all sufficient (in disagreement with WO 01-22972) for strong stimulation. For example, ODN IMT 028 and IMT 544, which have two TGs and two TTTT, have very poor activity.
g) The presence of TG dinucleotides plus the presence of the tetranucleotide TTTT, plus the presence of more than 25% Ts, is not at all sufficient (in disagreement with WO 01-22972) for strong stimulation. For example, ODN IMT 028 and IMT 546, which have two TGs, two TTTT and more than 25% Ts, have very poor activity.
h) Some Ts may be relevant for the activity of non CpG ODNs, but they are not necessarily related to a TG dinucleotide or to a TTTT motif. For example, the replacement of 2 Ts in ODN IMT 540, which are not involved in any TG or TTTT motif, is strongly detrimental (ODN 545). It is worth noting that both, ODN IMT 540 and IMT 545 have more than 25% T and do not have any TTTT or TG motifs. It is also worth noting that the substitution, in the poorly immunostimulatory ODN IMT 545 of two Ts, that generates two TGs dinucleotides, two TTTT motifs and increases the total content of Ts (ODN IMT 544) does not increase the activity (in disagreement with WO 01-22972).
i) The presence of the tetranucleotide CCCC and/or more than 50% of Cs is not at all sufficient (in disagreement with WO 01-22972) for a strong immunostimulation. For example, ODNs IMT 023, which have two CCCC and 63% of C, have very poor activity.
j) Replacement of some Ts for Cs or Gs in some T-rich oligonucleotides increases the activity (in disagreement with WO 01-22972). For example, replacement of 3 Ts in ODN IMT 059 by Cs (ODN IMT 037) increases the activity in a statistically significant manner. Therefore, the presence of Cs combined with some Ts may be relevant to achieve strong immunostimulation.
k) In general, T-rich oligonucleotides may have some inmunostimulatory activity in a phosphorothioate backbone, but are inactive in a phosphodiester backbone (in disagreement with WO 01-22972). For example, Table 5 shows that IMT 053, which consists of a polyT chain 24 nucleotides long, is able to stimulate the secretion of IL6 in a phosphothioate backbone, but not in a phosphodiester backbone. The same is true for ODN IMT 021, which contains more than 25% T and 6 TG dinucleotides. These two ODNs are also unable, in a phosphodiester backbone, to stimulate the expression of the activation markers CD 86, CD 40 and MHC class I on CD 19 positive (B) cells (Fig. 4).
l) In general, immunostimulatory oligonucleotides in a phosphodiester backbone are inactive as a double strand (in disagreement with WO 01-22972). For example, the strong stimulatory ODNs 2006 and IMT 504 are inactive as a double strand (not shown). Similar results were described in mice in the following publication: Zelenay, S., Elias, F. and Flo, J. Eur. J. Immunol. 33: 1382-92 (2003).

Taking into account all these facts, it was investigated which one is the minimal sequence necessary and sufficient that should be present in an non-CpG oligonucleotide in order to obtain a strong immunostimulatory activity, in a phosphodieter (natural) as well as in modified backbones.

**Table 5**

| Induction of IL6 secretion by phosphorothioate or phosphodiester | | | | |
|---|---|---|---|---|
| PyNTTTTGT ODNs | | | | |
| | | | | |
| | | *IL-6 Secretion* | | |
| | | *(pg*/*ml)* | | |
| ODN (O) | *Sequence* | *Avg.* | *N* | *SD* |
| IMT 022 (S) | TGCTGCAAAAGAGCAAAAGAGCAA | 38 | 4 | 17 |
| IMT 022 (O) | TGCTGCAAAAGAGCAAAAGAGCAA | 27 | 4 | 12 |
| IMT 053 (S) | TTTTTTTTTTTTTTTTTTTTTTTT | 123 | 4 | 21 |
| IMT 053 (O) | TTTTTTTTTTTTTTTTTTTTTTTT | 24 | 4 | 24 |
| IMT 021 (S) | TGCTGCTTTTGTGCTTTTGTGCTT | 182 | 4 | 51 |
| IMT 021 (O) | TGCTGCTTTTGTGCTTTTGTGCTT | 35 | 4 | 12 |
| IMT 504 (S) | TCATCATTTTGTCATTTTGTCATT | 285 | 4 | 39 |
| IMT 504 (O) | TCATCATTTTGTCATTTTGTCATT | 210 | 4 | 43 |
| IMT 506 (S) | TCTTCTTTTTGTCTTTTTGTCTTT | 228 | 4 | 70 |
| IMT 506 (O) | TCTTCTTTTTGTCTTTTTGTCTTT | 250 | 4 | 32 |
| 2006 (S) | TCGTCGTTTTGTCGTTTTGTCGTT | 216 | 4 | 84 |
| 2006 (O) | TCGTCGTTTTGTCGTTTTGTCGTT | 228 | 4 | 40 |
| None | --- | 28 | 4 | 14 |

| | | | | |
|---|---|---|---|---|
| IMT 021 (SEQ. ID N° 1); IMT 022 (SEQ. ID N° 8); IMT 053 (SEQ. ID N°19); IMT 504 (SEQ ID N°2); IMT 506 (SEQ ID N°4) (S): phosphorothioate. (O): phosphodiester. Phosphorothioate ODNs were added (6 µg/ml) at time 0 of incubation. Phosphodiester ODNs were added as follows: 30 µg/ml at time 0, 30 µg/ml after 4 hr and 30 µg/ml after 16 hr of incubation. | | | | |

The analysis of hundreds of ODNs (not shown) allowed definition of a core sequence *(motif)* responsible for the strong immunostimulatory activity of non-CpG ODNs as measured by B cell proliferation and IL6 secretion. This *motif* is the following:

**X₁X₂X₃X₄X₅X₆X₇X₈,**

wherein X₁ is C or T
wherein X₂ is C,T,G or A;
wherein X₃ is T or A;
wherein X₄ is T, C or G;
wherein X₅ is T, C or G;
wherein X₆ is T or G;
wherein X₇ is G;
wherein X₈ is T
wherein at least two of X₃, X₄, X₅ and X₆ are Ts
Table 6 shows the effect of changes in each of the nucleotides of the two non-CpG motifs present in the ODN IMT 504 (motif: CATTTTGT). Replacement of the C in position 1 of the motif by A (ODN IMT 531) or G (ODN IMT 533) resulted in a loss of about 50% in the activity. However, replacement of this C by T (ODN IMT 532) did not change the activity. Thus, in order to obtain maximal activity the first position of the motif should be occupied by a pyrimidine nucleotide (C or T). In position 2 of the motif, A (ODN IMT 504) or T (ODN IMT 535) or C (ODN IMT 534) are equivalent options.

In order to study the influence of a G nucleotide in position 2 of the motif without introduction of a CpG dinucleotide, ODNs with a T in the first position of the motif were synthesized (Table 7). As can be observed, any nucleotide in the second position of the motif is equivalent.

Replacement of the G in position 7 of the motif by A (ODN IMT 541), T (ODN IMT 542) or C (ODN IMT 543) is detrimental (Table 5). Thus, in order to obtain maximal activity position 7 of the motif should be preferably G.

Regarding the positions of the motif occupied by Ts, the most sensitive is the one in position 8. Replacement of this T for A (ODN IMT543) or C (ODN IMT599) or G (ODN IMT 604) is detrimental. Thus, in order to obtain maximal activity, position 8 of the motif should be preferably T. In position 3 of the motif the T could be replaced by A (ODN IMT IMT537) without activity loss. However, replacement of this T by C (ODN IMT595) or G (ODN IMT600) is detrimental. Thus, in order to obtain maximal activity, position 3 of the motif should be preferably T or A. In position 4 of the motif the T could be replaced by C (ODN IMT IMT596) or G (ODN IMT601) without significant activity loss. However, replacement of this T by A (ODN IMT538) is detrimental. Thus, in order to obtain maximal activity, position 4 of the motif should be preferably T or C or G. In position 5 of the motif, the T could be replaced by C (ODN IMT IMT597) or G (ODN IMT602) without significant activity loss. However, replacement of this T by A (ODN IMT539) is detrimental. Thus, in order to obtain maximal activity, position 5 of the motif should be preferably T or C or G. In position 6 of the motif, the T could be replaced by G (ODN IMT IMT603) without significant activity loss. However, replacement of this T by A (ODN IMT540) is detrimental. Thus, in order to obtain maximal activity, position 6 of the motif should be preferably T or G.

Simultaneous replacement of T by A in position 3 of the motif and T by G in position 6 of the motif did not result in any loss of activity (ODN IMT 608). However, in this case and in order to obtain maximal activity, positions 4 and 5 should be occupied by Ts (see ODNs IMT609, IMT610, IMT611, IMT612 and IMT614).

Replacement of two or more of the Ts by As within the motif results in more than 70% loss of activity (ODNs IMT 545, IMT 546, IMT 547, IMT 548, IMT 549, IMT 550, IMT 551 and IMT 552).

Taking these results into consideration, it can also be concluded that in order to obtain a strong immunostimulatory activity, two or more of the positions 3,4,5 and 6 of the motif should be occupied by Ts.

**TABLE 6**

| Induction of peripheral white blood cell proliferation bv phosphorothioate non-CpG | | | | |
|---|---|---|---|---|
| oligonucleotides of this invention derived from ODN IMT 504 | | | | |
| *ODN (S)* | *SEQUENCE (5'-3')* | *PROLIFERATION INDEX* | | |
| | | *(ODN at 0.375 µg*/*ml)* | | |
| | | *Avg.* | *N* | *SD* |
| 2006 | TCGTCGTTTTGTCGTTTTGTCGTT | 13.35 | 16 | 2.23 |
| IMT 022 | T**GC**T**GCAAAA**G**AGCAAAA**G**AGCAA** | 1.34 | 24 | 0.52 |
| IMT 504 | TCATCATTTTGTCATTTTGTCATT | 13.85 | 16 | 4.36 |
| IMT 531 | TCAT**A**ATTTTGT**A**ATTTTGTCATT | 7.59 | 12 | 2.71 |
| IMT 532 | TCA**TT**ATTTTGT**T**ATTTTGTCATT | 13.80 | 12 | 3.41 |
| IMT 533 | TCAT**G**ATTTTGT**G**ATTTTGTCATT | 6.20 | 12 | 2.71 |
| IMT 534 | TCATC**C**TTTTGTC**C**TTTTGTCATT | 11.67 | 12 | 4.49 |
| IMT 535 | TCATC**TT**TTTGTC**T**TTTTGTCATT | 12.49 | 12 | 2.51 |
| IMT 537 | TC**A**TC**AA**TTTGTC**AA**TTTGTCATT | 14.31 | 12 | 4.03 |
| IMT 595 | TCATCA**C**TTTGTCA**C**TTTGTCATT | 9.16 | 15 | 2.30 |
| IMT 600 | TCATCA**G**T**T**TGTCA**G**TTTGTCATT | 7.38 | 12 | 2.69 |
| IMT 538 | TCATCA**TA**TTGTCAT**A**TTGTCATT | 7.80 | 16 | 3.38 |
| IMT 596 | TCATCAT**C**TTGTCAT**CTT**GTCATT | 12.61 | 20 | 3.37 |
| IMT 601 | TCATCAT**G**TTGTCAT**G**TTGTCATT | 11.47 | 8 | 1.35 |
| IMT 539 | TCATCATT**A**TGTCAT**TA**TGTCATT | 5.63 | 16 | 3.52 |
| IMT 597 | TCATCATTCTGTCATT**C**TGTCATT | 13.98 | 20 | 3.78 |
| IMT 602 | TCATCATT**G**TGTCATT**G**TGTCATT | 13.23 | 20 | 3.47 |
| IMT 540 | TCA**T**CATTT**A**GTCATTT**A**GTCATT | 9.62 | 16 | 3.47 |
| IMT 603 | TCATCATTT**G**GTCATTT**G**GTCATT | 13.91 | 20 | 3.35 |
| IMT 541 | TCATCATTTT**A**TCATTT**TA**TCATT | 9.30 | 12 | 2.20 |
| IMT 542 | TCATCATTTT**T**TCATTTT**T**TCATT | 9.78 | 12 | 2.60 |
| IMT 543 | TCATCATTTT**C**TCATTTT**C**TCATT | 4.75 | 12 | 2.25 |
| IMT 544 | TCATCATTTTG**A**CATTTTG**A**CATT | 4.27 | 12 | 1.30 |
| IMT 599 | TCATCATTTTG**C**CATTTTG**C**CATT | 5.39 | 16 | 1.98 |
| IMT 604 | TCATCATTTTG**G**CATTTTG**G**CATT | 7.60 | 16 | 2.63 |
| IMT 608 | TCATCA**A**TT**G**GTCA**A**TT**G**GTCATT | 12.98 | 12 | 2.22 |
| IMT 609 | TCATCA**AA**TGGTCA**AA**TGGTCATT | 8.58 | 12 | 1.53 |
| IMT 610 | TCATCA**A**CTGGTCA**AC**T**G**GTCATT | 9.07 | 12 | 1.95 |
| IMT 611 | TCATCA**AG**TGGTCA**AG**TGGTCATT | 5.97 | 12 | 1.79 |
| IMT 612 | TCATCA**ATAG**GTCA**ATAG**GTCATT | 3.15 | 12 | 0.75 |
| IMT 614 | TCATCA**ATGG**GTCA**A**T**GG**GTCATT | 5.18 | 12 | 1.54 |
| IMT 545 | TCATCATTT**A**G**A**CA**T**TT**A**G**A**CATT | 4.19 | 12 | 1.81 |
| IMT 546 | TCATCATT**A**TG**A**CATT**A**TG**A**CATT | 2.90 | 12 | 1.16 |
| IMT 547 | TCATCAT**A**TTG**A**CAT**A**TTG**A**CATT | 3.34 | 12 | 1.16 |
| IMT 548 | TCATCATT**AA**G**A**CATT**AA**G**A**CATT | 3.40 | 12 | 1.53 |
| IMT 549 | TCATCAT**A**T**A**G**A**CAT**A**T**A**G**A**CATT | 3.51 | 12 | 1.03 |
| IMT 550 | TCATCAT**AA**TG**A**CAT**AA**TG**A**CATT | 1.91 | 12 | 0.71 |
| IMT 551 | TCATCA**TAAA**G**A**CAT**AAA**G**A**CATT | 2.17 | 12 | 0.72 |
| IMT 552 | TCATC**AAAAA**G**A**CA**AAAA**G**A**CATT | 1.37 | 12 | 0.83 |

| | | | | |
|---|---|---|---|---|
| IMT 022 (SEQ. ID N° 8); IMT 504 (SEQ ID N°2); IMT 531 (SEQ ID N°14); IMT 532 (SEQ ID N°15); IMT 533 (SEQ ID N°16); IMT 533 (SEQ ID N°17); IMT 534 (SEQ ID N°18); IMT 535 (SEQ ID N°19); IMT 537 (SEQ ID N°30); IMT 538 (SEQ ID N°31); IMT 539 (SEQ ID N°32); IMT 540 (SEQ ID N°33); IMT 541 (SEQ ID N°34); IMT 542 (SEQ ID N°35); IMT 543 (SEQ ID N°36); IMT 544 (SEQ ID N°37); IMT 545 (SEQ ID N°38); IMT 546 (SEQ ID N°39); IMT 547 (SEQ ID N°40); IMT 548 (SEQ ID N°41); IMT 549 (SEQ ID N°42); IMT 550 (SEQ ID N°43); IMT 551 (SEQ ID N°44); IMT 552 (SEQ ID N°45); IMT 539 (SEQ ID N°82); IMT 597 (SEQ ID N°83); IMT 602 (SEQ ID N°84); IMT 540 (SEQ ID N°85); IMT 603 (SEQ ID N°87); IMT 544 (SEQ ID N°88); IMT 599 (SEQ ID N°89); IMT 604 (SEQ ID N°90); IMT 608 (SEQ ID N°75); IMT 609 (SEQ ID N°76); IMT 610 (SEQ ID N°77); IMT 611 (SEQ ID N°78); IMT 612 (SEQ ID N°79); IMT 614 (SEQ ID N°81). | | | | |

**TABLE 7**

| Induction of peripheric white blood cell proliferation and IL-6 secretion bv | | | | | | | |
|---|---|---|---|---|---|---|---|
| Phosphorothioate non-CpG oligonucleotides with changes in the second | | | | | | | |
| position of the immunostimulatory motif | | | | | | | |
| | | | | | | | |
| *ODN (S)* | *SEQUENCE (5'-3')* | *PROLIFERATION INDEX* | | | *IL-6 (pg*/*ml)* | | |
| | | *(ODN at 0.375 µg*/*ml)* | | | *(ODN at 6.0 µg*/*ml)* | | |
| | | *Avg.* | *N* | SD | *Avg.* | N | *SD* |
| | | | | | | | |
| IMT 532 | TCAT**T**ATTTTGTTATTTTGTCATT | 10.10 | 4 | 1.61 | 221,7 | 4 | 35,3 |
| IMT 197 | TCAT**TT**TTTTGT**TT**TTTTGTCATT | 10.84 | 4 | 1.06 | 386,1 | 4 | 123,3 |
| IMT 198 | TCAT**TG**TTTTGT**TG**TTTTGTCATT | 10.00 | 4 | 1.75 | 235,3 | 4 | 27,1 |
| IMT 199 | TCAT**TC**TTTTGT**TC**TTTTGTCATT | 12.36 | 3 | 1.71 | 235,0 | 4 | 6,0 |

| | | | | | | | |
|---|---|---|---|---|---|---|---|
| IMT 532 (SEQ ID N°15); IMT 197 (SEQ. ID N° 10); IMT 198 (SEQ ID N°11); IMT 199 (SEQ ID N°12) | | | | | | | |

To investigate the effect of changes in the position of the motif within the ODN chain, the non-CpG motif CATTTTGT present in ODN IMT504 was introduced into different locations in a 24-nucleotide long poly T chain (Fig. 5). As can be seen, the poly T ODN (S) by itself had a significant mitogenic activity. On the other hand, introduction of only one CATTTTGT motif resulted in an increment of 1.2 to 1.8 times (measured by proliferation assays), or 2.4 to 3.5 times (measured by IL-6 secretion assays) in the immunostimulatory activity of the polyT chain, depending on where the motif was located. A distance of at least two nucleotides from the 5' end or four from the 3' end seems to be necessary in order to reach maximal activity (ODNs IMT174 to IMT 179). Introduction of two motifs in optimal positions (ODN IMT182) resulted in an increment of 1.9 times (measured by proliferation assays), or 2.9 times (measured by IL-6 secretion assays) in the immunostimulatory activity of the polyT chain. This indicates that the contribution of the second motif is negligible. On the other hand, the activity of the most effective ODNs like ODN IMT504 is more than 3 times larger than the activity of the poly T as measured by proliferation assays, a fact which suggests that there is also a significant influence of the composition of at least some of the nucleotides surrounding the motif of this invention on the overall activity of the ODN.

### EXAMPLE 4

### Effect of structure modification on the immunostimulatory activity of non-CpG oligonucleotides of this invention: influence of the nucleotide composition outside of the active motif.

Results shown in Fig. 5 indicate that composition of the oligonucleotide outside of the non-CpG core motif is important in order to reach optimal immunostimulatory activity. Therefore, a number of oligonucleotides were synthesized with changes in the composition of the nucleotides surrounding the motifs. Table 8 shows the effect of changes in the composition of the first four nucleotides of the 5' end and of the last four nucleotides of the 3' end in the immunostimulatory activity of ODN IMT 504. As can be observed, the best choices are: C or G in position -1 respect to the two motifs, C, T or G in position -2, any nucleotide in positions -3 and -4, A or T in position +1, G in position +2, any nucleotide in positions +3 and G in position +4 .

Of course, other nucleotide combinations not represented in this table may have equal or even better effect on the activity of the non-CpG immunostimulatory oligonucleotides. One of ordinary skill in the art can empirically determine other effective combinations.

**TABLE 8**

| Induction of peripheral white blood cell proliferation by non- CpG ODN(S) IMT | | | | | | | |
|---|---|---|---|---|---|---|---|
| 504 with variations in the composition outside of the two immunostimulatory motifs | | | | | | | |
| *ODN (S)* | *SEQUENCE (5'-3')* | *PROLIFERATION INDEX* | | | *IL-6 (pg*/*ml)* | | |
| | | *(ODN at 0.375 µq*/*ml)* | | | *(ODN at 6.0 µg*/*ml)* | | |
| | | *Avg.* | N | *SD* | *Avg.* | N | *SD* |
| | | | | | | | |
| IMT 552 | TGCTGCAAAAGAGCAAAAGAGCAA | 1,9 | 12 | 0,7 | <39,9 | 4 | ---- |
| IMT 504 | TCATCATTTTGTCATTTTGTCATT | 11,0 | 8 | 0,9 | 171 | 2 | 14 |
| IMT 559 | ACATCATTTTGTCATTTTGTCATT | 13,4 | 4 | 1,0 | 128 | 3 | 3 |
| IMT 560 | CCATCATTTTGTCATTTTGTCATT | 12,1 | 4 | 0,9 | 145 | 4 | 32 |
| IMT 561 | GCATCATTTTGTCATTTTGTCATT | 9,5 | 4 | 1,1 | 209 | 4 | 74 |
| IMT 562 | TAATCATTTTGTCATTTTGTCATT | 12,4 | 4 | 1,5 | 171 | 4 | 24 |
| IMT 563 | TTATCATTTTGTCATTTTGTCATT | 11,1 | 3 | 0,7 | 172 | 4 | 63 |
| IMT 564 | TGATCATTTTGTCATTTTGTCATT | 12,8 | 4 | 0,9 | 118 | 4 | 38 |
| IMT 565 | TCCTCATTTTGTCATTTTGTCATT | 14,1 | 4 | 0,5 | 135 | 4 | 25 |
| IMT 566 | TCTTCATTTTGTCATTTTGTCATT | 13,9 | 4 | 2,2 | 157 | 4 | 28 |
| IMT 567 | TCGTCATTTTGTCATTTTGTCATT | 12,9 | 4 | 0,7 | 259 | 4 | 25 |
| IMT 568 | TCAACATTTTGTCATTTTGTCATT | 10,3 | 4 | 1,1 | 153 | 4 | 35 |
| IMT 569 | TCACCATTTTGTCATTTTGTCATT | 15,0 | 4 | 1,2 | 199 | 3 | 12 |
| IMT 570 | TCAGCATTTTGTCATTTTGTCATT | 12,5 | 4 | 0,4 | 181 | 3 | 2 |
| IMT 571 | TCATCATTTTGTCATTTTGTAATT | 14,2 | 4 | 0,7 | 163 | 4 | 26 |
| IMT 572 | TCATCATTTTGTCATTTTGTTATT | 13,2 | 4 | 1,4 | 182 | 3 | 67 |
| IMT 573 | TCATCATTTTGTCATTTTGTGATT | 8,7 | 4 | 0,7 | 177 | 4 | 29 |
| IMT 574 | TCATCATTTTGTCATTTTGTCCTT | 8,1 | 4 | 0,7 | 179 | 3 | 58 |
| IMT 575 | TCATCATTTTGTCATTTTGTCTTT | 10,8 | 4 | 0,8 | 150 | 3 | 47 |
| IMT 576 | TCATCATTTTGTCATTTTGTCGTT | 12,6 | 4 | 1,1 | 202 | 4 | 55 |
| IMT 577 | TCATCATTTTGTCATTTTGTCAAT | 10,0 | 4 | 1,4 | 243 | 3 | 8 |
| IMT 578 | TCATCATTTTGTCATTTTGTCACT | 10,9 | 4 | 0,9 | 213 | 2 | 9 |
| IMT 579 | TCATCATTTTGTCATTTTGTCAGT | 10,7 | 4 | 1,7 | 182 | 4 | 18 |
| IMT 580 | TCATCATTTTGTCATTTTGTCATA | 11,7 | 4 | 1,4 | 194 | 4 | 36 |
| IMT 581 | TCATCATTTTGTCATTTTGTCATC | 10,7 | 4 | 1,0 | 173 | 4 | 18 |
| IMT 582 | TCATCATTTTGTCATTTTGTCATG | 12,1 | 4 | 0,8 | 242 | 3 | 231 |

| | | | | | | | |
|---|---|---|---|---|---|---|---|
| IMT 552 (SEQ. ID N° 45); IMT 504 (SEQ ID N°2); IMT 559 (SEQ ID N°46); IMT 560 (SEQ. ID N° 47); IMT 561 (SEQ ID N°48); IMT 562 (SEQ ID N°49); IMT 563 (SEQ ID N°50); IMT 564 (SEQ ID N°51); IMT 565 (SEQ ID N°52); IMT 566 (SEQ ID N°53); IMT 567 (SEQ ID N°54); IMT 568 (SEQ ID N°55); IMT 569 (SEQ ID N°56); IMT 570 (SEQ ID N°57); IMT 571 (SEQ ID N°58); IMT 572 (SEQ ID N°59); IMT 573 (SEQ ID N°60); IMT 574 (SEQ ID N°61); IMT 575 (SEQ ID N°62); IMT 576 (SEQ ID N°63); IMT 577 (SEQ ID N°64); IMT 578 (SEQ ID N°65); IMT 578 (SEQ ID N°66); IMT 580 (SEQ ID N°67); IMT 581 (SEQ ID N°68); IMT 582 (SEQ ID N°69) ., | | | | | | | |

### EXAMPLE 5

### Effect of structure modification on the immunostimulatory activity of non-CpG oligonucleotides of this invention: influence of the size of the oligonucleotide

Table 9 shows that ODNs with one immunostimulatory motif are active if the chain is 16 or more nucleotides long. Activity is maximal if the ODN is between 20 and 25 nucleotides long. *All the ODNs of within this range bearing at least one non-CpG motif of this invention assayed during this study (more than hundred) were very active.*

**TABLE 9**

| Effect of the size of phosphorothioate non-CpG oligonucleotides on B cells | | | | | | | |
|---|---|---|---|---|---|---|---|
| proliferation and IL6 secretion | | | | | | | |
| | | | | | | | |
| *ODN (S)* | *SEQUENCE (5'-3')* | *PROLIFERATION INDEX* | | | *IL-6 (pg*/*ml)* | | |
| | | *(ODN at 0.375 µg*/*ml)* | | | *(ODN at 6.0 µg*/*ml)* | | |
| | | *Avg.* | *N* | *SD* | *Avg.* | *N* | *SD* |
| IMT 187 | TTTTCATTTTGT | 0.56 | 8 | 0.20 | <50 | 4 | - |
| IMT 188 | TTTTCATTTTGTTTTT | 2.76 | 8 | 1.73 | 147 | 4 | 40 |
| IMT 189 | TTTTCATTTTGTTTTTTTTT | 8.21 | 8 | 3.19 | 227 | 4 | 21 |
| IMT 175 | TTTTCATTTTGTTTTTTTTTTTTT | 8.92 | 8 | 1.80 | 220 | 4 | 18 |
| IMT 179 | TTTTTTTTTTTTCATTTTGTTTTT | 6.06 | 8 | 2.00 | 224 | 4 | 82 |
| IMT 191 | TTTTCATTTTGTTTTTTTTTTTTTTTTT | 6.64 | 8 | 2.37 | 205 | 4 | 15 |

| | | | | | | | |
|---|---|---|---|---|---|---|---|
| IMT 187 (SEQ ID N°70); IMT 188 (SEQ ID N°71); IMT 189 (SEQ ID N°72); IMT 179 (SEQ ID N°73); IMT 191 (SEQ ID N°77) | | | | | | | |

### EXAMPLE 6

### Induction of peripheral white blood cell IgM secretion by phosphorothioate non-CpG oligonucleotides of this invention

Induction of IgM, secretion in peripheral white blood cells is another important marker of immunostimulatory activity of oligonucleotides. Table 10 shows the stimulation of IgM secretion by several of the phosphorothioate non-CpG oligonucleotides described above. As can be observed, the most active non-CpG ODN(S)s in induction of proliferation and IL6 secretion are also the best in induction of IgM secretion.

**TABLE 10**

| Induction of peripheral white blood cell IgM secretion bv phosphorothioate | | | | |
|---|---|---|---|---|
| non-CpG oligonucleotides of this invention | | | | |
| *ODN (S)* | *SEQUENCE (5'-3')* | *IgM SECRETION (ng*/*ml)* | | |
| | | *(ODN at 1.50 µg*/*ml)* | | |
| | | *Avg.* | *N* | *SD* |
| cells alone | | 76 | 9 | 50 |
| 2006 | TCGTCGTTTTGTCGTTTTGTCGTT | 778 | 47 | 204 |
| IMT 503 | TTGTTGTTTTGTTGTTTTGTTGTT | 640 | 19 | 220 |
| IMT 504 | TCATCATTTTGTCATTTTGTCATT | 912 | 24 | 244 |
| IMT 505 | TCCTCCTTTTGTCCTTTTGTCCTT | 664 | 23 | 336 |
| IMT 506 | TCTTCTTTTTGTCTTTTTGTCTTT | 751 | 43 | 237 |
| IMT 509 | AAAAAACTAAAAAAAACTAAAAAA | 195 | 17 | 77 |

| | | | | |
|---|---|---|---|---|
| IMT 504 (SEQ ID N°2); IMT 505 (SEQ ID N°3); IMT 506 (SEQ ID N°4); IMT 503 (SEQ ID N°7); IMT 509 (SEQ ID N°13) | | | | |

### EXAMPLE 7

### Stimulation of the expression of CD40, MHC I and MHC II in B lymphocyte by phosphorothioate non-CpG oligonucleotides of this invention

Human PMBC were incubated with ODN IMT 504, ODN 2006 as a positive control and ODN IMT 022 as a negative control (Fig 6). As can be observed, ODN IMT 504 is as active as the CpG ODN 2006 for stimulation of the expression of CD40, MHC I and MHC II on B lymphocytes.

### EXAMPLE 8

### Stimulation of purified B lymphocytes by non-CpG oligonucleotides of this invention

Human CD19⁺ (B) cells were purified to more than 95% purity. Table 11 and Fig. 7 show that the immunostimulatory activity on these purified cells is comparable to the one observed using human PMBC. These results indicate that stimulation by the non-CpG oligonucleotides of this invention on human cells is direct.

**TABLE 11**

| Induction of proliferation, IL6 and IgM secretion on purified B cells by | | | | | | | | | | |
|---|---|---|---|---|---|---|---|---|---|---|
| phosphorothioate non-CpG oligonucleotides of this invention | | | | | | | | | | |
| | | | | | | | | | | |
| *ODN (S)* | *SEQUENCE (5'-3')* | *Proliferation Index* | | | *IL-6 (pg*/*ml)* | | | *IgM (nglml)* | | |
| | | *(ODN at 0.375 µg*/*ml)* | | | *(ODN at 6.0 µg*/*ml)* | | | *(ODN at 1.5 µg*/*ml)* | | |
| | | **Ave**. | **N** | **SD** | **Ave**. | **N** | **SD** | **Ave**. | **N** | **SD** |
| IMT 022 | TGCTGCAAAAGAGCAAAAGAGCAA | 1,28 | 16 | 0,80 | 1019 | 5 | 665 | 469 | 7 | 182 |
| IMT 504 | TCATCATTTTGTCATTTTGTCATT | 47,95 | 16 | 6,41 | 11002 | 5 | 884 | 1274 | 9 | 252 |
| IMT 506 | TCTTCTTTTTGTCTTTTTGTCTTT | 25,58 | 16 | 7,84 | 10933 | 3 | 1628 | 1083 | 10 | 430 |
| 2006 | TCGTCGTTTTGTCGTTTTGTCGTT | 60,82 | 24 | 9,79 | 7631 | 6 | 997 | 1292 | 12 | 245 |

| | | | | | | | | | | |
|---|---|---|---|---|---|---|---|---|---|---|
| IMT 022 (SEQ. ID N° 8); IMT 504 (SEQ ID N°2); IMT 506 (SEQ ID N°4) | | | | | | | | | | |

### EXAMPLE 9

### Immunostimulation by phosphodiester non-CpG oligonucleotides of this invention

Fig 8 and Table 12 show the effect of phosphodiester non-CpG oligonucleotides of this invention on human PMBC. Since phosphodiester oligonucleotides are very sensitive to nucleases they were added to the culture three times (0, 4, and 16 hr) to a final concentration of 30 µg/ml. A very potent phosphodiester CpG ODN (ODN 2080) was used as positive control in cytometric assays (Hartmann G, Weeratna R, Ballas ZK, Payette P, Suparto, I, Rasmussen WL, Wadschmidt M, Sajuthi D, Purcells RH, Davis HL, Krieg AM. Delineation of a CpG phosphorothioate oligodeoxynucleotide for activating primate immune responses in vitro and in vivo. J. Immunol.164, 1617-1624, (2000)). As can be observed, under these conditions, phosphodiester ODNs bearing non-CpG motifs of this invention have immunostimulatory activity. Regarding this, it is worth noting the differences of these non-CpG ODNs with those non-CpG ODNs claimed in WO 01/22972 (see table 5 and Fig 4).

**TABLE 12**

| Immune-stimulation by *phosphodiester* non-CpG oligonucleotides of this invention | | | | |
|---|---|---|---|---|
| *ODN (O)* | *SEQUENCE (5'-3')* | *IL-6 Secretion (pg*/*ml)* | | |
| | | *(OON at 6.0 µg*/*ml)* | | |
| | | *Avg.* | *N* | *SD* |
| IMT 022 | TGCTGCAAAAGAGCAAAAGAGCAA | 23 | 8 | 10 |
| IMT 053 | TTTTTTTTTTTTTTTTTTTTTTTT | 24 | 4 | 24 |
| IMT 504 | TCATCATTTTGTCATTTTGTCATT | 207 | 6 | 46 |
| IMT 506 | TCTTCTTTTTGTCTTTTTGTCTTT | 403 | 3 | 220 |
| 2006 | TCGTCGTTTTGTCGTTTTGTCGTT | 228 | 4 | 40 |

| | | | | |
|---|---|---|---|---|
| IMT 022 (SEQ. ID N° 8); IMT 504 (SEQ ID N°2); IMT 053 (SEQ ID N°19); IMT 506 (SEQ ID N°4) | | | | |

### EXAMPLE 10

### Induction of cell proliferation in peripheral white blood cells of monkeys of the species Cebus apella and Macaca fascicularis by non-CpG ODN(S)s of this invention

Non-CpG ODN(S)s of this invention are not very effective immunostimulants in mice, pigs and sheep. However, they are effective in monkeys. For example, Fig.9 shows the immunostimulatory activity of non-CpG ODN(S)s on peripheral white blood cells of monkeys of the species *Cebus apella* and *Macaca fascicularis.* According to these results the most effective non-CpG ODNs in non human primates are those bearing the CATTTTGT motif. Therefore, monkeys can be used as animal models for research on clinical applications of these non-CpG ODN(S)s.

### EXAMPLE 11

### Vaccination of subjects

Vaccine formulations containing the non-CpG ODNs adjuvant of this invention can be used to vaccinate subjects against a variety of bacterial and viral disease agents and against tumor cells. Table 13 shows the effect of inoculation of monkeys of the species *Cebus apella* with a vaccine formulation that includes recombinant Hepatitis B surface antigen (rHBsAg) and alumina in presence or absence of ODN IMT 504 as adjuvant.

**TABLE 13**

| Anti-HBs responses in *Cebus apella* immunized against HBsAg with IMT | | | | |
|---|---|---|---|---|
| ODNs | | | | |
| | | | | |
| *ODN* (*S*) | *Pre- prime* | *Post- prime* | | *Post- boost* |
| | | *14 days* | *28 days* | *14 days* |
| None | 0 | 0,4 ± 0,1 | 14 ± 20 | 190±52 |
| IMT 504 | 0 | 10 ± 14 | 113 ± 114 | 659±296 |
| 2006 | 0 | 22 ± 24 | 67 ± 57 | 705±315 |

As can be observed, there is a dramatic increment in the title of anti-HBsAg in animals vaccinated with HBsAg plus IMT ODN(S) 504 as compared with those vaccinated with HBsAg alone. CpG ODN 2006 has a performance as adjuvant similar to the non-CpG ODNs of this invention.

In particular, a human can be vaccinated against hepatitis B by administration of a vaccine formulation that includes recombinant Hepatitis B surface antigen (rHBsAg) and alumina and one or more of the oligonucleotides of this invention as adjuvants. The amount of rHBsAg in each dose, and the administration schedule, can vary according to the age of the human. For example, for humans from about birth to about 12 years old, a three-dose schedule of about 2.5 µg to about 5 µg of rHBsAg can be administered at 0, 1-3 months afterward and 4-18 months afterward, preferably at 0, 2 and 6 months. One or more of the oligonucleotides of this invention can be present in the formulation from about 10 µg to about 1,000 µg per dose.

For humans from about 12 to about 60 years old, a three-dose schedule of about 5 µg to about 40 µg of rHBsAg can be administered at 0, 1-3 months afterward and 4-18 months afterward, preferably at 0, 2 and 6 months. One or more of the oligonucleotides of this invention can be present in the formulation from about 10 µg to about 1,000 µg per dose.

### EXAMPLE 12

### Stimulation of the expression of costimulatory molecules in malignant B-lymphocytes by phosphorothioate non-CpG oligonucleotides

The stimulation of malignant B lymphocytes recovered from blood of patients suffering from chronic lymphocytic leukemia (CLL) by phosphorothioate non-CpG oligonucleotides of this invention was examined by flow cytometric (FACS) analysis. The results of a typical FACS analysis are shown in Fig. 10. As can be seen, the phosphorothioate non-CpG oligonucleotide IMT 504 of this invention is able to stimulate expression of CD86, CD40 and MHC class I surface costimulatory molecules on malignant B lymphocytes so well as the CpG ODN 2006.

### EXAMPLE 13

### Stimulation of malignant B cell apoptosis by phosphorothioate non-CpG oligonucleotides

The stimulation of apoptosis in malignant B lymphocytes recovered from blood of patients suffering from chronic lymphocytic leukemia (CLL) by phosphorothioate non-CpG oligonucleotides of this invention was examined by flow cytometric (FACS) analysis using as apoptosis markers propidium iodide and annexin V. The result of a tipical FACS analysis are shown in Fig 11. As can be observed, the non CpG oligonucleotide IMT 504 of this invention is able to increase the apoptosis of the leukemia cells so well as the CpG ODN 2006.

### EXAMPLE 14

### Stimulation of the expression of costimulatory molecules on plasmacytoid dendritic cells by phosphorothioate non-CpG oligonucleotides of this invention

The stimulation of plasmacytoid dendritic cells recovered from blood of normal donors by phosphorothioate non-CpG oligonucleotides of this invention was examined by flow cytometric (FACS) analysis. The results of a typical FACS analysis are shown in Fig. 12. As can be seen, the phosphorothioate non-CpG oligonucleotide IMT 504 of this invention is able to stimulate expression of CD86, CD40 and MHC class I surface molecules on plasmacytoid dendritic cells so well as the CpG ODN 2006.

Table 14 shows that the non-CpG oligonucleotides of this invention are also able to stimulate purified human plasmacytoid dendritic cells to secrete interferon alpha if the oligonucleotide backbone is phosphodiester.

**TABLE 14**

| Interferon alpha secretion by purified plasmacytoid dendritic cells incubated with | | | | |
|---|---|---|---|---|
| phosphorothioate and *phosphodiester* non-CpG oligonucleotides of this invention | | | | |
| | | *IFN Alpha Secretion* | | |
| *ODN* | *SEQUENCE (5'-3')* | *(pg*/*ml*) | | |
| | | *Avg.* | *N* | *SD* |
| IMT 022 (S) | TGCTGCAAAAGAGCAAAAGAGCAA | <31 | 3 | -- |
| IMT 504 (S) | TCATCATTTTGTCATTTTGTCATT | <31 | 3 | -- |
| IMT 022 (O) | TGCTGCAAAAGAGCAAAAGAGCAA | <31 | 3 | --- |
| IMT 504 (O) | TCATCATTTTGTCATTTTGTCATT | 2300 | 3 | 632 |

| | | | | |
|---|---|---|---|---|
| IMT 022 (SEQ. ID N° 8); IMT 504 (SEQ ID N°2). Phosphorothioate ODNs were added (1.5 µg/ml) at time 0 of incubation. Phosphodiester ODNs were added as follows: 30 µg/ml at time 0, 30 µg/ml after 4 hr and 30 µg/ml after 16 hr of incubation. | | | | |

### EXAMPLE 15

### Treatment of subjects with tumoral disease

Pharmaceutical formulations containing one or more of the immunostimulatory oligonucleotides of this invention, can be used to treat subjects against a variety of tumoral diseases. In particular, a human with a Melanoma can be treated by administration of a pharmaceutical formulation containing the oligonucleotide of this invention as the active component. The amount of oligonucleotide in each dose, and the administration schedule, can vary according to the corporal mass of the subject and stage in tumor progression. For example, for a human of about 70 kg who has an advanced, unresectable metastatic melanoma, a dose of about 1 mg of the oligonucleotide of this invention can be administered 3 times per week for about 10 weeks.

### EXAMPLE 16

### Induction of peripheral white blood cell IL-10 secretion and inhibition of IL-5 secretion by phosphorothioate non-CpG oligonucleotides of this invention

IL-10 is a potent suppressor of IgE production and induction and maintenance of epitope-specific T cell anergy is associated with increased IL-10. Fig 13 shows the stimulation of IL-10 secretion by the phosphorothioate non-CpG oligonucleotide IMT 504 and controls in PMBCs of several donors. As can be observed, IMT 504 is as active as the CpG positive control ODN 2006. Allergen-specific Th2 cells produce IL-5, which promotes the differentiation, activation and survival of eosinophils. Fig 14 shows that the phosphorothioate non-CpG oligonucleotide IMT 504 and the CpG positive control ODN 2006 are repressors of the production of IL-5 in several human PMBCs induced by IL-4 + Con A.

### EXAMPLE 17

### Treatment of subjects with allergic disease

Pharmaceutical formulations containing one or more of the immunostimulatory oligonucleotides of this invention, can be used to treat subjects against a variety of allergic diseases.

In particular, a human with asthma can be treated by administration in the respiratory tract of an aerosolized pharmaceutical formulation containing the oligonucleotide of this invention as the active component. The amount of oligonucleotide in each dose, and the administration schedule, can vary according to the subject.

### Oligonucleotide sequences which are outside the scope of the invention

The following oligonucleotide sequences (disclosed in the above listed prior art publications) are excluded from the scope of the claims:
ACTTCTCATAGTCCTTTGGTCCAG
TTGTTTTTTTGTTTTTTTGTTTTT
TTTTTTTTGTTTTTTTGTTTTT
TTTTGGGGTTTTGGGGTTTT
CTGGTCTTTCTGGTTTTTTTCTGG
CTGTGCTTTCTGTGTTTTTCTGTG
GAACCTTCCATGCTGTT
GCTGAACCTTCCATGCTGTT
TTTTTGTTTTGTGGTTTTGTGGTT
TTGGTTTTTTTGGTTTTTTTTTGG
ACATCTGGTTCTTACTTCAGG

GGTTCTTTTGGTCCTTGTCT
GGTTCTTTTGGTCCTTATCT
TGGTCTTTTGGTCCTTGTCT
TTCAGTTGTCTTGCTGCTTAGCTAA
GGTTCTTTTGGTCCTTGTCT
TGGTGGTTTTGTGGTTTTGTGGTT

TCCTCCTTTTGTCCTTTTGTCCTT
CTGTGCTTTCTGTGTTTTTCTGTG
TGCTGCTTGTGCTTGTGCTT
GGTTCTTTTGGTCCTTGTCT
CTGGTCTTTCTGGTTTTTTTCTGG
TNGTNGTTTTGTNGTTTTGTNGTT
CTGTGCTTTCTGTGTTTTTCTGTG
GAACCTTCCATGCTGTT
GCTGAACCTTCCATGCTGTT
TTTTTGTTTTGTGGTTTTGTGGTT

TTCAGTTGTCTTGCTGCTTAGCTAA
TTGGTTTTTTTGGTTTTTTTTTGG
ACATCTGGTTCTTACTTCAGG
GGTTCTTTTGGTCCTTATCT
TGGTCTTTTGGTCCTTGTCT

TTGTTGTTGTTGTTTGTTGTTGTTG
TCCATGTNGTTCCTGATGCT
ACTTCTCATAGTCCCTTTGGTCCAG
TTGTTTTTTTGTTTTTTTGTTTTT
TTTTTTTTGTTTTTTTGTTTTT 
CTTAGGGGTGTTCTTAGGTGGTAACCCCTAGGGGTTACCACCTTCATTG
GAAAACCTT
 GAAAACCTT
TTAGTTGTTAGTTATTAGTT
TTAGTTGTTAGTTTTTAGTT
TTAGTTGTTAGTTCTTAGTT
TTAGTTGTTAGTTGTTAGTT
GGCTGAGCTGGCTTTTTCTTGTGG
CTGGTCTTTCTGGTTTTTTTCTGG
CTCCTATTGGTGGTTTCCTAT
CTCCTATTGGGTTTTTCCTAT
CTCCTATTGGTGTTTTCCTAT
CTCCTATTGGTTGTTTCCTAT
CTCCTATTTGGTGTTTCCTAT
CTCCTATTTGTGGTTTCCTAT
CTCCTATTTTGGGTTTCCTAT
TTGGAGTTTTTGGTGGGG
TTGGAGTTTTTGGTTTTT
CTGATGTGTTGAAGAACA
TGCTGTATTTCTGGTACA
GTTATCCCTGCTGT
ACCTTGCTGTACTG
CTGGCTTTTGGGTT
TCTGTTGTGACTCAAG

### SEQUENCE LISTING

(1) GENERAL INFORMATION:
   (i) APPLICANT: IMMUNOTECH SA
   (ii) TITLE OF INVENTION: Immunostimulatory oligonucleotides and uses thereof.
   (iii) NUMBER OF SEQUENCES: 74
   (iv) CORRESPONDENCE ADDRESS:
      (A) ADDRESSEE: Dragotti & Associati
      (B) STREET: Galleria San Babila 4/C
      (C) CITY: Milano
      (D) STATE:
      (E) COUNTRY: Italy
      (F) ZIP: 20122
   (v) COMPUTER READABLE FORM:
      (A) MEDIUM TYPE: Floppy disk
      (B) COMPUTER: IBM PC compatible
      (C) OPERATING SYSTEM:
      (D) SOFTWARE:
   (vi) CURRENT APPLICATION DATA:
      (A) APPLICATION NUMBER:
      (B) FILING DATE:
      (C) CLASSIFICATION:
   (vii) PRIORITY APPLICATION DATA:
      (A) APPLICATION NUMBER: CA-2388049
      (B) FILING DATE: 2002-05-30
   (viii) ATTORNEY/AGENT INFORMATION:
      (A) NAME: Roberto Pistolesi
      (B) REGISTRATION NUMBER: 94880
      (C) REFERENCE/DOCKET NUMBER: 03mg06e
   (ix) TELECOMMUNICATION INFORMATION:
      (A) TELEFONE: +39 02 799340
      (B) TELEFAX: +39 02 784427
(2) INFORMATION FOR SEQ ID N°1 (IMT 021):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 1:
      TGCTGCTTTTGTGCTTTTGTGCTT
(2) INFORMATION FOR SEQ ID N°2 (IMT 504):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
(ii) MOLECULE TYPE: DNA
(iii) SEQUENCE DESCRIPTION: SEQ ID N°2:
   TCATCATTTTGTCATTTTGTCATT
(2) INFORMATION FOR SEQ ID N°3 (IMT 505):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 3:
      TCCTCCTTTTGTCCTTTTGTCCTT
(2) INFORMATION FOR SEQ ID N°4 (IMT 506):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 4:
      TCTTCTTTTTGTCTTTTTGTCTTT
(2) INFORMATION FOR SEQ ID N°5 (IMT 501):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 5:
      TAGTAGTTTTGTAGTTTTGTAGTT
(2) INFORMATION FOR SEQ ID N°6 (IMT 502):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 6:
      TGGTGGTTTTGTGGTTTTGTGGTT
(2) INFORMATION FOR SEQ ID N°7 (IMT 503):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 7:
      TTGTTGTTTTGTTGTTTTGTTGTT
(2) INFORMATION FOR SEQ ID N°8 (IMT 022):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 8:
      TGCTGCAAAAGAGCAAAAGAGCAA
(2) INFORMATION FOR SEQ ID N°9 (IMT 023):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 9: .
      TGCTGCCCCCGCGCCCCCGCGCCC
(2) INFORMATION FOR SEQ ID N°10 (IMT 197):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 10:
      TCATTTTTTTGTTTTTTTGTCATT
(2) INFORMATION FOR SEQ ID N°11 (IMT 198):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 11:
      TCATTGTTTTGTTGTTTTGTCATT
(2) INFORMATION FOR SEQ ID N°12 (IMT 199):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 12:
      TCATTCTTTTGTTCTTTTGTCATT
(2) INFORMATION FOR SEQ ID N°13 (IMT 509):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 13:
      AAAAAACTAAAAAAAACTAAAAAA
(2) INFORMATION FOR SEQ ID N°14 (IMT 531):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 14:
      TCATAATTTTGTAATTTTGTCATT
(2) INFORMATION FOR SEQ ID N°15 (IMT 532):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 15:
      TCATTATTTTGTTATTTTGTCATT
(2) INFORMATION FOR SEQ ID N°16 (IMT 533):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 16:
      TCATGATTTTGTGATTTTGTCATT
(2) INFORMATION FOR SEQ ID N°17 (IMT 534):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 17:
      TCATCCTTTTGTCCTTTTGTCATT
(2) INFORMATION FOR SEQ ID N°18 (IMT 535):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 18:
      TCATCTTTTTGTCTTTTTGTCATT
(2) INFORMATION FOR SEQ ID N°19 (IMT 053):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 19:
      TTTTTTTTTTTTTTTTTTTTTTTT
(2) INFORMATION FOR SEQ ID N°20 (IMT 173):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 20:
      CATTTTGTTTTTTTTTTTTTTTTT
(2) INFORMATION FOR SEQ ID N°21. (IMT 174):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 21:
      TTCATTTTGTTTTTTTTTTTTTTT
(2) INFORMATION FOR SEQ ID N°22 (IMT 175):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 22:
      TTTTCATTTTGTTTTTTTTTTTTT
(2) INFORMATION FOR SEQ ID N°23 (IMT 176):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 23:
      TTTTTTCATTTTGTTTTTTTTTTT
(2) INFORMATION FOR SEQ ID N°24 (IMT 177):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 24:
      TTTTTTTTCATTTTGTTTTTTTTT
(2) INFORMATION FOR SEQ ID N°25 (IMT 178):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 25:
      TTTTTTTTTTCATTTTGTTTTTTT
(2) INFORMATION FOR SEQ ID N°26 (IMT 179):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 26:
      TTTTTTTTTTTTCATTTTGTTTTT
(2) INFORMATION FOR SEQ ID N°27(IMT 180):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 27:
      TTTTTTTTTTTTTTCATTTTGTTT
(2) INFORMATION FOR SEQ ID N°28 (IMT 181):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 28:
      TTTTTTTTTTTTTTTTCATTTTGT
(2) INFORMATION FOR SEQ ID N°29 (IMT 182):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 29:
      TTTTCATTTTGTCATTTTGTTTTT
(2) INFORMATION FOR SEQ ID N°30 (IMT 537):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 30:
      TCATCAATTTGTCAATTTGTCATT
(2) INFORMATION FOR SEQ ID N°31 (IMT 538):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ 10 N° 31:
      TCATCATATTGTCATATTGTCATT
(2) INFORMATION FOR SEQ ID N°32 (IMT 539):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 32:
      TCATCATTATGTCATTATGTCATT
(2) INFORMATION FOR SEQ ID N°33 (IMT 540):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 33:
      TCATCATTTAGTCATTTAGTCATT
(2) INFORMATION FOR SEQ ID N°34 (IMT 541):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 34:
      TCATCATTTTATCATTTTATCATT
(2) INFORMATION FOR SEQ ID N°35 (IMT 542):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 35:
      TCATCATTTTTTCATTTTTTCATT
(2) INFORMATION FOR SEQ ID N°36 (IMT 543):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 36:
      TCATCATTTTCTCATTTTCTCATT
(2) INFORMATION FOR SEQ ID N°37 (IMT 544):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 37:
      TCATCATTTTGACATTTTGACATT
(2) INFORMATION FOR SEQ ID N°38 (IMT 545):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N°38:
      TCATCATTTAGACATTTAGACATT
(2) INFORMATION FOR SEQ ID N°39 (IMT 546):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N° 39:
      TCATCATTATGACATTATGACATT
(2) INFORMATION FOR SEQ ID N°40 (IMT 547):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N°40:
      TCATCATATTGACATATTGACATT
(2) INFORMATION FOR SEQ ID N°41 (IMT 548):
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA
   (iii) SEQUENCE DESCRIPTION: SEQ ID N°41:
      TCATCATTAAGACATTAAGACATT
(2) INFORMATION FOR SEQ ID N°42 (IMT 549):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 42:
      TCATCATATAGACATATAGACATT
(2) INFORMATION FOR SEQ ID N°43 (IMT 550):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°43:
      TCATCATAATGACATAATGACATT
(2) INFORMATION FOR SEQ ID N°44 (IMT 551):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°44:
      TCATCATAAAGACATAAAGACATT
(2) INFORMATION FOR SEQ ID N°45 (IMT 552):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°45:
      TCATCAAAAAGACAAAAAGACATT
(2) INFORMATION FOR SEQ ID N°46 (IMT 559):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°46:
      ACATCATTTTGTCATTTTGTCATT
(2) INFORMATION FOR SEQ ID N°47 (IMT 560):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 47:
      CCATCATTTTGTCATTTTGTCATT
(2) INFORMATION FOR SEQ ID N°48 (IMT 561):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°48:
      GCATCATTTTGTCATTTTGTCATT
(2) INFORMATION FOR SEQ ID N°49 (IMT 562):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°49:
      TAATCATTTTGTCATTTTGTCATT
(2) INFORMATION FOR SEQ ID N°50 (IMT 563):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 50:
      TTATCATTTTGTCATTTTGTCATT
(2) INFORMATION FOR SEQ ID N°51 (IMT 564):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°51:
      TGATCATTTTGTCATTTTGTCATT
(2) INFORMATION FOR SEQ ID N°52 (IMT 565):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°52:
      TCCTCATTTTGTCATTTTGTCATT
(2) INFORMATION FOR SEQ ID N°53 (IMT 566):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°53:
      TCTTCATTTTGTCATTTTGTCATT
(2) INFORMATION FOR SEQ ID N°54 (IMT 567):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 54:
      TCGTCATTTTGTCATTTTGTCATT
(2) INFORMATION FOR SEQ ID N°55 (IMT 568):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°55:
      TCAACATTTTGTCATTTTGTCATT
(2) INFORMATION FOR SEQ ID N°56 (IMT 569):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°56:
      TCACCATTTTGTCATTTTGTCATT
(2) INFORMATION FOR SEQ ID N°57 (IMT 570):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°57:
      TCAGCATTTTGTCATTTTGTCATT
(2) INFORMATION FOR SEQ ID N°58 (IMT 571):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°58:
      TCATCATTTTGTCATTTTGTAATT
(2) INFORMATION FOR SEQ ID N°59 (IMT 572):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°59:
      TCATCATTTTGTCATTTTGTTATT
(2) INFORMATION FOR SEQ ID N°60 (IMT 573):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°60:
      TCATCATTTTGTCATTTTGTGATT
(2) INFORMATION FOR SEQ ID N°61 (IMT 574):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°61:
      TCATCATTTTGTCATTTTGTCCTT
(2) INFORMATION FOR SEQ ID N°62 (IMT 575):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 62:
      TCATCATTTTGTCATTTTGTCTTT
(2) INFORMATION FOR SEQ ID N°63 (IMT 576):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 63:
      TCATCATTTTGTCATTTTGTCGTT
(2) INFORMATION FOR SEQ ID N°64 (IMT 577):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°64:
      TCATCATTTTGTCATTTTGTCAAT
(2) INFORMATION FOR SEQ ID N°65 (IMT 578):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°65:
      TCATCATTTTGTCATTTTGTCACT
(2) INFORMATION FOR SEQ ID N°66 (IMT 579):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°66:
      TCATCATTTTGTCATTTTGTCAGT
(2) INFORMATION FOR SEQ ID N°67 (IMT 580):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°67:
      TCATCATTTTGTCATTTTGTCATA
(2) INFORMATION FOR SEQ ID N°68 (IMT 581):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 68:
      TCATCATTTTGTCATTTTGTCATC
(2) INFORMATION FOR SEQ ID N°69 (IMT 582):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°69:
      TCATCATTTTGTCATTTTGTCATG
(2) INFORMATION FOR SEQ ID N°70 (IMT 187):
   (A) SEQUENCE CHARACTERISTICS:
      (A) LENGTH: 12 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°70:
      TTTTCATTTTGT
(2) INFORMATION FOR SEQ ID N°71 (IMT 188):
   (A) SEQUENCE CHARACTERISTICS:
      (A) LENGTH: 16 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°71:
      TTTTCATTTTGTTTTT
(2) INFORMATION FOR SEQ ID N°72 (IMT 189):
   (A) SEQUENCE CHARACTERISTICS:
      (A) LENGTH: 20 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 72:
      TTTTCATTTTGTTTTTTTTT
(2) INFORMATION FOR SEQ ID N°73 (IMT 179):
   (A) SEQUENCE CHARACTERISTICS:
      (A) LENGTH: 24 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N° 73:
      TTTTTTTTTTTTCATTTTGTTTTT
(2) INFORMATION FOR SEQ ID N°74 (IMT 191):
   (A) SEQUENCE CHARACTERISTICS:
      (A) LENGTH: 28 base pairs
      (B) TYPE: nucleic acid
      (C) STRANDEDNESS: single
      (D) TOPOLOGY: linear
   (B) MOLECULE TYPE: DNA
   (C) SEQUENCE DESCRIPTION: SEQ ID N°74:
      TTTTCATTTTGTTTTTTTTTTTTTTTTT

## Claims

1. An immunostimulatory oligonucleotide having up to 100 nucleotides and comprising a non-palindromic nucleic acid sequence motif having the following formula:
TCATCATTTTGTCATTTTGTCATT (SEQ ID N°2).

2. A immunostimulatory oligonucleotide according to claim 1, **characterized by** having up to 40 nucleotides.

3. A immunostimulatory oligonucleotide which is:
TCATCATTTTGTCATTTTGTCATT (SEQ ID N°2).

4. The immunostimulatory oligonucleotide of claims 1-3 wherein the immunostimulatory oligonucleotide is encapsulated in a slow release delivery vehicle.

5. The immunostimulatory oligonucleotide of claims 1-3 wherein the immunostimulatory oligonucleotide is included in a pharmaceuticallly acceptable carrier.

6. A pharmaceutical composition comprising an immunostimulatory oligonucleotide according to claims 1-3 and a pharmaceutically acceptable carrier.

7. A composition according to claim 6 wherein said immunostimulatory oligonucleotide is included in a plasmid.

8. A composition according to claim 6 further comprising an antigen.

9. A composition according to claim 8 wherein the antigen is selected from the group consisting of viruses, bacteria, fungi, parasites, tumoral cells, toxins, allergens, proteins, glycolipids and polysaccharides.

10. A composition according to claim 8 wherein the antigen is a viral antigen, a bacterial antigen, a human or animal tumor cell and/or a fungal antigen.

11. A composition according to claim 8 wherein the antigen is encoded by a plasmid.

12. A composition according to claims 6-11 which is in liquid or lyophilized form.

13. Use of an immunostimulatory oligonucleotide according to claims 1-5 for the manufacture of a medicament for treating, preventing or ameliorating a tumoral disease.

14. The use of claim 13, wherein said tumoral disease is selected from Chronic Myelogenous Leukemia, Precursor B-lymphoblastic lymphoma, B-cell chronic lymphocytic leukaemia, Lymphoplasmacytic lymphoma, Mantle cell lymphoma, Follicle center lymphoma (follicular and diffuse), Marginal zone-B lymphoma, Extranodal lymphoma, Nodal marginal zone B-cell lymphoma, Splenic marginal zone B-cell lymphoma, Hairy cell leukaemia, Plasmocytoma, Diffuse large B-cell lymphoma, Burkitt's lymphoma, High grade B-cell lymphoma, Burkitt like, Melanoma, Kaposi's Sarcoma, Multiple Myeloma, Renal Cell Carcinoma, Bladder Cancer, Lung Cancer, Skin Cancer, Breast Cancer, Colon Cancer and Uterus Cancer.

15. Use of an immunostimulatory oligonucleotide according to claims 1-5 for the manufacture of a medicament for treating, preventing or ameliorating an immunological disorders selected from Allergy, Severe Combined Immunodeficiency, Chronic Granulomatous disease, and Acquired Immunodeficiency Disease.

16. The use of claims 13-15, wherein the medicament is to be administered to a human.

17. The use of claims 13-15, wherein the oligonucleotide has been conjugated with one or more antigens.

18. The use of claims 13-15, wherein the medicament comprises an antigen.

19. The use of claims 13-15, wherein the medicament is to be administered by intramuscular, oral, intranasal, anal, vaginal or transdermic route.

## Patentansprüche

1. Immunstimulatorisches Oligonukleotid mit bis zu 100 Nukleotiden und welches ein nicht palindromisches Nukleinsäuresequenzmotiv mit folgender Formel umfasst:
TCATCATTTTGTCATTTTGTCATT (SEQ ID N°2).

2. Immunstimulatorisches Oligonukleotid nach Anspruch 1, dadurch gekenntzeichnet, dass es bis zu 40 Nukleotide hat.

3. Immunstimulatorisches Oligonukleotid, das:
TCATCATTTTGTCATTTTGTCATT (SEQ ID N° 2) ist.

4. Immunstimulatorisches Oligonukleotid nach den Ansprüchen 1-3, wobei das immunstimulatorische Oligonukleotid in einem Liefer-Vehikel mit langsamer Freisetzung eingekapselt ist.

5. Immunstimulatorisches Oligonukleotid nach den Ansprüchen 1-3, wobei das immunstimulatorische Oligonukleotid in einem pharmazeutisch annehmbaren Träger beinhaltet ist.

6. Pharmazeutische Zusammensetzung, welche ein immunstimulatorisches Oligonukleotid nach den Ansprüchen 1-3 und einen pharmazeutisch annehmbaren Träger umfasst.

7. Zusammensetzung nach Anspruch 6, wobei das genannte immunitimulatorische Oligonukleotid in einem Plasmid beinhaltet ist.

8. Zusammensetzung nach Anspruch 6, welche zusätzlich ein Antigen umfasst.

9. Zusammensetzung nach Anspruch 8, wobei das Antigen aus der Gruppe gewählt wird, die aus Viren, Bakterien, Pilze, Schädlinge, Tumorzellen, Toxine, Allergene, Proteine, Glycolipide und Polysaccharide besteht.

10. Zusammensetzung nach Anspruch 8, wobei das Antigen ein virales Antigen, ein bakterielles Antigen, eine menschliche oder tierische Tumorzelle und/oder ein Pilz-Antigen ist.

11. Zusammensetzung nach Anspruch 8, wobei das Antigen durch ein Plasmid kodiert wird.

12. Zusammensetzung nach den Ansprüchen 6-11, welche in flüssiger oder lyophilierter Form ist.

13. Verwendung eines immunstimulatorischen Oligonukleotids nach den Ansprüchen 1-5, für die Herstellung eines Medikamentes zur Behandlung, Vorbeugung oder Verbesserung einer Geschwulstkrankheit.

14. Verwendung nach Anspruch 13, wobei die genannte Geschwulstkrankheit aus Folgende gewählt wird:
chronische myeloische Leukämie, 6-Zell-Vorläufer lymphoblastisches Lymphom, B-Zell chronisch lymphatische Leukämie, lymphoplasmazytoides Lymphom, Mantelzell-Lymphom, Follikelzentrums-Lymphom (follikulär und diffus), Marginalzonen B-Zell Lymphom, extranodales Lymphom, nodales Marginalzonen B-Zell Lymphom, Marginalzonen B-Zell Lymphom der Milz, Haarzell-Leukämie, Plasmozytom, diffuses großzelliges B-Zell Lymphom, Burkitt-Lymphom, hochmalignes B-Zell Lymphom, Burkitt-ähnlich, Melanom, Kaposi Sarkom, multiples Myelom, Nierenzellkarzinom, Blasenkrebs, Lungenkrebs, Hautkrebs, Brustkrebs, colorektaler Krebs und Gebahrmutterkrebs.

15. Verwendung eines immunstimulatorischen Oligonukleotids nach den Ansprüchen 1-5, für die Herstellung eines Medikamentes zur Behandlung, Vorbeugung oder Verbesserung einer immunologischen Störung, die aus Folgende gewählt wird: Allergie, schwerer kombinierter Zmmuridefekt, chronisch granulomatöse Krankheit und erworbenes Immundefekt-Syndrom.

16. Verwendung nach den Ansprüchen 13-15, wobei das Medikament zur menschlichen Verabreichung vorgesehen ist.

17. Verwendung nach den Ansprüchen 13-15, wobei das Oligonukleotid mit einem oder mehreren Antigenen konjugiert wurde.

18. Verwendung nach den Ansprüchen 13-15, wobei das Medikament ein Antigen umfasst.

19. Verwendung nach den Ansprüchen 13-15, wobei das Medikament zur intramuskulären, oralen, intranasalen, analen, vaginalen oder transkutanen Verabreichung vorgesehen ist.

## Revendications

1. Oligonucléotide immunostimulateur ayant jusqu'à 100 nucléotides et comprenant un motif de séquence non-palindromique d'acide nucléique ayant la formule suivante :
TCATCATTTTGTCATTTTGTCATT (SEQ ID N° 2).

2. Oligonucléotide immunostimulateur selon la revendication 1, **caractérisé en ce qu'**il a jusqu'à 40 nucléotides.

3. Oligonucléotide immunostimulateur qui est :
TCATCATTTTGTCATTTTGTCATT (SEQ ID N° 2).

4. Oligonucléotide immunostimulateur selon les revendications 1-3, dans lequel l'oligonucléotide immunostimulateur est capsulé dans un véhicule à libération lente.

5. Oligonucléotide immunostimulateur selon les revendications 1-3, dans lequel l'oligonucléotide immunostimulateur est inclus dans un porteur pharmaceutiquement acceptable.

6. Composition pharmaceutique comprenant un oligonucléotide immunostimulateur selon les revendications 1-3 et un porteur pharmaceutiquement acceptable.

7. Composition selon la revendication 6 dans laquelle ledit oligonucléotide immunostimulateur est inclus dans un plasmide.

8. Composition selon la revendication 6, comprenant en outre un antigène.

9. Composition selon la revendication 8 dans laquelle l'antigène est choisi parmi le groupe consistant en des virus, des bactéries, des champignons, des parasites, des cellules tumorales, des toxines, des allergènes, des protéines, des glycolipides et des polysaccharides.

10. Composition selon la revendication 8, dans laquelle l'antigène est un antigène viral, un antigène bactérien, une cellule tumorale humaine ou animale et/ou un antigène fongique.

11. Composition selon la revendication 8 dans laquelle l'antigéne est codé par un plasmide.

12. Composition selon les revendications 6-11 qui est sous forme liquide ou lyophilisée.

13. Utilisation d'un oligonucléotide immunostimulateur selon les revendications 1-5 pour l'élaboration d'un médicament pour traiter, prévenir ou améliorer une maladie tumorale.

14. Utilisation selon la revendication 13, dans laquelle ladite maladie tumoral est choisi parmi la leucémie myélogène chronique, lymphome lymphoblastique à précurseur B, leucémie lymphotique chromique à cellules B, lymphome lymphoplasmocytique (folliculaire et diffus) lymphome de la zone marginale B, lymphome extranodal, lymphome de la zone marginale nodale à cellules B, lymphome de la zone marginale splénique à cellules B, leucémie à cellules chevelues, plasmocytome, lymphome diffus à grandes cellules B, lymphome de Burkitt, lymphome à cellules B de haut degré, de type Burkitt, mélanome, sarcome de Kaposi, myélome multiple, carcinome à cellules rénales, cancer du poumon, cancer de la peau, cancer du serin, cancer du colon et cancer de l'utérus.

15. Utilisation d'un oligonucléotide immunostimulateur selon les revendications 1-5 pour l'élaboration d'un médicament pour traiter, prévenir ou améliorer des troubles immunologiques choisis parmi l'allergie, l'immuriodéficierice combinée sévère, la maladie granulomateuse chronique, et le syndrome d'immunodéficience acquise.

16. Utilisation selon les revendications 13-15, dans laquelle le médicament doit être administré à un être humain.

17. Utilisation selon les revendications 13-15, dans laquelle l'oligonucléotide a été conjugué à un ou plusieurs antigènes.

18. Utilisation selon les revendications 13-15, dans laquelle le médicament comprend un antigène.

19. Utilisation selon les revendications 13-15, dans laquelle le médicament doit être administré par voie intramusculaire, orale, intranasale, anale, vaginale ou transdermique.
